# EUROPEAN PATENT SPECIFICATION

(11) **EP 4 267 220 B1**
(45) Date of publication and mention of the grant of the patent: **15.04.2026**
(21) Application number: 21831312.0
(22) Date of filing: 10.12.2021
(51) Int. Cl.: A61M 5/32, A61M 5/24

(54) **MECHANISMS FOR RECEIVING AN INJECTOR**
MECHANISMEN ZUR AUFNAHME EINES INJEKTORS
MÉCANISMES DE RÉCEPTION D'UN INJECTEUR

(30) Priority: 24.12.2020 EP 20217175
(43) Date of publication of application: 01.11.2023
(73) Proprietor: SHL Medical AG, 6302 Zug (CH)
(72) Inventor: HUANG, Chun-Hsien, Taoyuan City (TW)
(86) International application number: PCT/EP2021/085130
(87) International publication number: WO 2022/135966

(56) References cited:
- EP-A1- 3 666 314

## Description

### Technical Field

The present disclosure generally relates to mechanisms for receiving an injector. In particular, a mechanism for receiving an injector having a needle and a needle shield covering the needle, where the mechanism comprises a shield holder movable between an aligned position and an offset position.

### Background

An autoinjector may comprise a spring, a lid, a needle guard and a housing in which an injector is arranged. The injector may comprise a medicament container with medicament, a needle and a needle shield covering the needle. A user may remove the lid together with the needle shield. The needle guard may then pop out to hide the exposed needle.

Most autoinjectors are for single use and therefore have a high environmental impact. Although multiple use autoinjectors exist, these do not provide sufficient protection against needlestick injuries, in particular when the user takes out the injector and tries to put back the needle cover on the injector.

Furthermore, a problem for both single use autoinjectors and multiple use autoinjectors is that the user may forget where the needle shield is put or may lose the needle shield. The user may for example drop the needle shield on the floor such that the needle shield rolls away. The user experience of such autoinjectors is therefore not always satisfying.

Patent application EP 3 666 314 A1 discloses an injection device comprising a cap actuation mechanism which comprises a movable gripper for holding and releasing the needle cap and an actuator to actuate the gripping and release function and to translate the gripper and therewith the needle cap from a position aligned with the needle to a position away from the injection path of the syringe.

### Summary

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially", "radial", "radially", "rotation", "rotational" and "rotationally" refer to a direction generally perpendicular to the longitudinal direction and at least partially extending around the longitudinal direction.

According to the invention, there is provided a mechanism for receiving an injector having a needle and a needle shield covering the needle, the mechanism comprising a housing having an injector holder for holding the injector; a shield holder for holding the needle shield, the shield holder being movable relative to the housing between an aligned position for holding the needle shield aligned with the longitudinal axis, and an offset position for holding the needle shield offset from the longitudinal axis; and a rotator rotatable around the longitudinal axis from a first rotator position to a second rotator position; wherein the mechanism is configured to cause a movement of the shield holder from the aligned position to the offset position by means of a movement of the rotator from the first rotator position to the second rotator position.

In the offset position, the needle shield is held by the shield holder such that the needle shield does not cover or obstruct the needle. A medicament delivery procedure can thereby be performed by the mechanism while the shield holder is in the offset position and holds the needle shield. When the injector is held by the injector holder and the shield holder is in the offset position, the mechanism can deliver medicament from the injector. The mechanism thus provides functionality of removing the needle shield from the needle and securely holding the needle shield while delivering medicament from the injector by means of the mechanism. The user can load an injector to the injector holder and remove the injector from the injector holder after medicament delivery from the injector by means of the mechanism.

The mechanism is configured to remove the needle shield from the needle mechanically, without needing any electronics, while the needle is protected by the mechanism before and after medicament delivery, and while the needle shield is safely held in the mechanism. The mechanism thus, enables a simple and safe use.

The mechanism according to the present disclosure may thus be a multiple use mechanism. The mechanism may be an autoinjector.

The housing may be generally cylindrical. Alternatively, or in addition, the housing may be centered with respect to the longitudinal axis.

The injector holder may be an injector seat. Alternatively, or in addition, the injector holder may be fixed in the housing. The injector holder may be configured to hold and support the injector such that the injector is oriented coaxially with the longitudinal axis.

Throughout the present disclosure, the injector may be a syringe. The injector may contain medicament.

The shield holder may be rotatable between the aligned position and the offset position, e.g. about a shield holder pivot. In this case, the shield holder may rotate about an axis parallel with, and offset from, the longitudinal axis. Thus, the shield holder may be arranged to move between the aligned position and the offset position substantially in a plane, or in a plane, perpendicular to the longitudinal axis.

The shield holder may be arranged radially inside the housing with respect to the longitudinal axis. The shield holder may be a shield seat.

The rotator may be cylindrical. Alternatively, or in addition, the rotator may be centered with respect to the longitudinal axis.

The rotator may be arranged to rotate 30 degrees to 180 degrees, such as 90 degrees, about the longitudinal axis from the first rotator position to the second rotator position. Alternatively, or in addition, the rotator may be arranged radially inside the housing with respect to the longitudinal axis. The rotator may be centered with respect to the longitudinal axis.

The mechanism may further comprise an actuating body movable relative to the housing along the longitudinal axis between a first actuating position and a second actuating position. The second actuating position may be proximal of the first actuating position.

The actuating body may further be configured to move along the longitudinal axis between the first actuating position and a third actuating position. In this case, the first actuating position may lie between the second actuating position and the third actuating position. Medicament delivery may in this case be effected by moving the actuating position from the first actuating position to the third actuating position. The actuating body may for example be pressed against an injection site such that the actuating body moves in the distal direction from the first actuating position to the third actuating position and such that the needle penetrates into the injection site.

The actuating body may be a tube. The actuating body may be centered with respect to the longitudinal axis.

The actuating body may be arranged at least partly inside the housing. Alternatively, or in addition, the actuating body may be arranged inside the rotator.

The mechanism may further comprise a needle guard. The needle guard may be integrally formed with the actuating body, e.g. at a proximal end of the actuating body.

The mechanism may further comprise a primary force device arranged to force the actuating body in the distal direction from the second actuating position back to the first actuating position. The primary force device may be a spring, such as a coil spring. Alternatively, or in addition, the primary force device may be arranged between the injector holder and the actuating body.

The mechanism may further comprise a secondary force device arranged to force the actuating body in the proximal direction from the third actuating position back to the first actuating position. The secondary force device may be a spring, such as a coil spring. Alternatively, or in addition, the secondary force device may be arranged between the housing and the actuating body.

The actuating body may cover the needle in each of the first actuating position and the second actuating position. The needle may be exposed when the actuating body is moved from the first actuating position to the third actuating position.

The mechanism may further comprise a guiding member for limiting a movement of the actuating body relative to the housing along the longitudinal axis. In this case, the guiding member may be arranged to move along the longitudinal axis relative to the housing and may be prevented from rotating about the longitudinal axis relative to the housing. To this end, the mechanism may comprise a one or more guiding grooves parallel with the longitudinal axis and a pin engaging each guiding groove. The guiding member may for example comprise a plurality of guiding grooves and the housing may comprise housing pins, each engaging one of the guiding grooves.

The guiding member may be a sleeve. Alternatively, or in addition, the guiding member may be centered with respect to the longitudinal axis.

The mechanism may further comprise a rotator force device arranged to force the rotator in a distal direction along the longitudinal axis. The rotator force device may be a spring, such as a coil spring. The rotator force device may be arranged between the guiding member and the rotator.

The rotator may be rotatable from the first rotator position to the second rotator position in response to one or more movements of the actuating body between the first actuating position and the second actuating position. Thus, the shield holder can be caused to move from the aligned position to the offset position by means of movements of the actuating body between the first actuating position and the second actuating position. The movements of the actuating body can be performed by a user by gripping the housing with one hand, gripping the actuating body with the other hand, and moving the actuating body relative to the housing.

The mechanism may further comprise a cam profile and at least one cam follower arranged to follow the cam profile. In this case, the rotator may be arranged to rotate from the first rotator position to the second rotator position by means of interaction between the at least one cam follower and the cam profile and by means of one or more movements of the actuating body between the first actuating position and the second actuating position.

The cam profile may be provided on the rotator. In this case, the cam profile may be formed by a distal surface of the rotator.

One or more of the at least one cam follower may be provided on the actuating body. Alternatively, or in addition, one or more of the at least one cam follower may be provided on the housing.

The actuating body may be rotatable relative to the housing about the longitudinal axis between an open body position for receiving the injector in the injector holder, and a closed body position for enclosing the received injector. The user can thereby twist the actuating body to open the mechanism for inserting the injector. After inserting the injector, the user can twist the actuating body in the opposite direction for closing the mechanism, e.g. by rotating the actuating body from the open body position to the closed body position. The rotation of the actuating body can be effected by gripping the actuating body with one hand, gripping the housing with the other hand, and twisting these parts about the longitudinal axis.

The housing may comprise a housing wall having an opening, and the actuating body may comprise a body wall. The housing wall may overlap the body wall and the body wall may be positioned between the housing wall and the injector holder when the actuating body is in the open body position. The body wall may cover the opening when the actuating body is in the closed body position. In this way, the mechanism can have a compact shape both when the actuating body is in the closed body position and in the open body position.

The injector holder may comprise a lifter movable between a raised position for raising the injector from the injector holder, and a lowered position for allowing the injector to be seated in the injector holder such that the injector is in an orientation concentric with the longitudinal axis. In the raised position, the injector can be better accessed for manual grabbing. In the lowered position, the injector is properly positioned in the injector holder for medicament delivery.

In the raised position, the lifter may raise the injector to a preliminary position. The injector holder and the lifter may be configured such that the injector is inclined relative to the longitudinal axis when the lifter is in the raised position. In the lowered position, the lifter may lower the injector to a ready-for-injection position.

The lifter may be movable from the lowered position to the raised position substantially in a lateral plane, or in a lateral plane, perpendicular to the longitudinal axis.

The lifter may be rotatable between the raised position and the lowered position about a lifter axis parallel with the longitudinal axis.

The mechanism may be configured to transmit a movement of the actuating body from the open body position to the closed body position to a movement of the lifter from the raised position to the lowered position. The actuating body may comprise a pushing structure arranged to push the injector when the actuating body moves from the open body position to the closed body position such that the lifter is forced from the raised position to the lowered position.

The pushing structure may comprise an edge extending in a direction perpendicular to the longitudinal axis.

The pushing structure may comprise a wall arranged in a plane perpendicular to the longitudinal axis.

The mechanism may further comprise a lifter force device arranged to force the lifter from the lowered position towards the raised position. The lifter force device may for example be a torsion spring. In this case, one end of the torsion spring may be connected to the lifter and one end of the torsion spring may be connected to a support body of the injector holder.

The mechanism further comprises at least one magnet. The shield is magnetically forced towards the rotator by means of the at least one magnet from the aligned position to the offset position during rotation of the rotator from the first rotator position to the second rotator position. The shield holder is forced to the offset position by means of magnetic attraction caused by the at least one magnet.

The rotator may comprise one magnet arranged on an inner surface of the rotator, and the shield holder may be made from a magnetic material, such as iron, cobalt, and/ or nickel, or an alloy made from at least one of a magnetic material.

The at least one magnet comprises one or more magnets on the rotator and one more magnets on the shield holder. The one or more magnets on the rotator may comprise at least two magnets symmetrically arranged on the inner surface of the rotator. Alternatively, the one or more magnets on the rotator may comprise at least three magnets distributed around the inner surface of the rotator to surround the shield holder. In the example that the rotator is arranged to rotate 90 degrees, the one or more magnets on the rotator comprises at least four magnets, so that the rotator rotates around 90 degrees to force the shield holder to move to the offset position; and a further 90 degrees rotation of the rotator forces the shield holder back to the aligned position.

The magnets on the rotator may have poles of different polarities facing the shield holder. For example, when the at least one magnet on the shield holder is rotationally aligned with one of the magnets on the rotator such that poles of different types are facing each other, the shield holder is forced to move from the aligned position to the offset position by means of magnetic attraction. Conversely, when the at least one magnet on the shield holder is rotationally aligned with one of the magnets on the rotator such that poles of the same type are facing each other, the shield holder is forced to move from the offset position to the aligned position by means of magnetic repulsion.

The rotator may be rotatable around the longitudinal axis from the second rotator position to a third rotator position. In this case, the shield holder may be magnetically forced away from the rotator by means of the magnets from the offset position to the aligned position when the rotator is in the third rotator position. The shield holder may be forced to the aligned position by means of magnetic repulsion caused by the at least one magnet. The mechanism according to this variant thus also provides functionality of realigning the needle shield with the needle for again covering the needle after delivery of medicament from the injector by means of the mechanism.

As an alternative or addition to the use of magnetic repulsion, the shield holder may be arranged to be magnetically attracted to the offset position and arranged to be forced to the aligned position by means of a spring, or vice versa. The spring may for example be a torsion spring.

The rotator may be rotatable from the first rotator position to the second rotator position in response to one or more movements of the actuating body between the first actuating position and the second actuating position.

The shield holder may be connected to the actuating body. In this case, the shield holder may be removed from the needle by movement of the actuating body from the first actuating position to the second actuating position. The shield holder may then be put back to the injector to again cover the needle by movement of the actuating body from the second actuating position to the first actuating position.

In this variant, the shield holder may be pivotally connected to the actuating body for rotation about an axis parallel with the longitudinal axis. A torsion spring may be arranged at the pivot.

The shield holder may be connected to the injector holder. In this variant, the shield holder may be pivotally connected to the injector holder for rotation about an axis offset from, and perpendicular with the longitudinal axis. A torsion spring may be arranged at the pivot.

The shield holder may comprise a stop arm movable from a stopping position where the stop arm prevents the shield holder from moving from the aligned position to the offset position, to a releasing position where the stop arm does not prevent the shield holder from moving from the aligned position to the offset position.

The stop arm may be arranged to follow the cam profile and move from the stopping position to the releasing position in response to rotation of the rotator about the longitudinal axis.

The shield holder may further comprise a holder arm and a holder spring arranged to force the holder arm in a proximal direction. In this case, the stop arm may be pivotally connected to the holder arm. The holder arm may be a lever arm. The holder spring may be connected between the injector holder and the holder arm. The holder arm may be arranged to pivot relative to the injector holder, e.g. about a pivot perpendicular to the longitudinal axis.

The shield holder may further comprise a shield seat for holding the needle shield. In this case, the holder arm may be arranged between the shield seat and the holder spring.

The mechanism may further comprise a flexible strap arranged to adopt a relaxed state between the injector and the injector holder when the injector holder holds the injector, and arranged to adopt a tensioned state for raising the injector relative to the injector holder substantially in a lateral direction, or in a lateral direction, perpendicular to the longitudinal axis, by manual pulling of the strap.

According to a further aspect, there is provided a mechanism for receiving an injector having a needle and a needle shield covering the needle, the mechanism comprising a housing; an injector holder for holding the injector, the injector holder being movable relative to the housing along a longitudinal axis from a loading position for loading the injector to the injector holder, to a delivery position at least partly inside the housing; and a cover detachably attached to the housing, the cover being arranged to engage the needle shield by movement of the injector holder from the loading position to the delivery position, and arranged to remove the needle shield from the needle by detachment of the cover from the housing.

The injector holder of this aspect may function as a drawer. When the injector holder is in the loading position, the injector holder may extend distally outside the housing.

The injector holder may be movable from the loading position to the delivery position in a proximal direction along the longitudinal axis.

### Brief Description of the Drawings

Further details, advantages and aspects of the present disclosure will become apparent from the following description taken in conjunction with the drawings, wherein:
Fig. 1: schematically represents a perspective view of a mechanism for receiving an injector;
Fig. 2: schematically represents a perspective exploded view of the mechanism and the injector;
Fig. 3: schematically represents a further perspective exploded view of the mechanism and the injector;
Fig. 4: schematically represents a perspective view of the mechanism when an actuating body is in an open body position;
Fig. 5: schematically represents a perspective view of the mechanism when receiving the injector;
Fig. 6: schematically represents a perspective view of an actuating body of the mechanism;
Fig. 7A: schematically represents a side view of the mechanism when the actuating body is in the open body position and in a first actuating position;
Fig. 7B: schematically represents a side view of the mechanism when the actuating body is in a closed body position;
Fig. 7C: schematically represents a side view of the mechanism when the actuating body is in a second actuating position and when a rotator is in a first rotator position;
Fig. 7D: schematically represents a side view of the mechanism when the rotator has rotated from the first rotator position;
Fig. 7E: schematically represents a side view of the mechanism when the actuating body is in the first actuating position and the rotator is in a second rotator position;
Fig. 7F: schematically represents a side view of the mechanism when the actuating body is in a third actuating position;
Fig. 8A: schematically represents a partial side view of the mechanism according to Fig. 7A;
Fig. 8B: schematically represents a partial side view of the mechanism according to Fig. 7B;
Fig. 8C: schematically represents a partial side view of the mechanism according to Fig. 7C;
Fig. 8D: schematically represents a partial side view of the mechanism according to Fig. 7D;
Fig. 8E: schematically represents a partial side view of the mechanism according to Fig. 7E;
Fig. 8F: schematically represents a partial side view of the mechanism according to Fig. 7F;
Fig. 9A: schematically represents a partial distal view of the mechanism according to Figs. 7A and 8A;
Fig. 9B: schematically represents a partial distal view of the mechanism according to Figs. 7B and 8B;
Fig. 9C: schematically represents a partial distal view of the mechanism according to Figs. 7C and 8C;
Fig. 9D: schematically represents a partial distal view of the mechanism according to Figs. 7D and 8D;
Fig. 9E: schematically represents a partial distal view of the mechanism according to Figs. 7E and 8E;
Fig. 9F: schematically represents a partial distal view of the mechanism according to Figs. 7F and 8F;
Fig. 10: schematically represents a cross-sectional proximal view of the mechanism in Figs. 7B, 8B and 9B;
Fig. 11A: schematically represents a cross-sectional side view of the mechanism according to Figs. 7A, 8A and 9A;
Fig. 12A: schematically represents a further cross-sectional side view of the mechanism according to Figs. 7A, 8A, 9A and 11A;

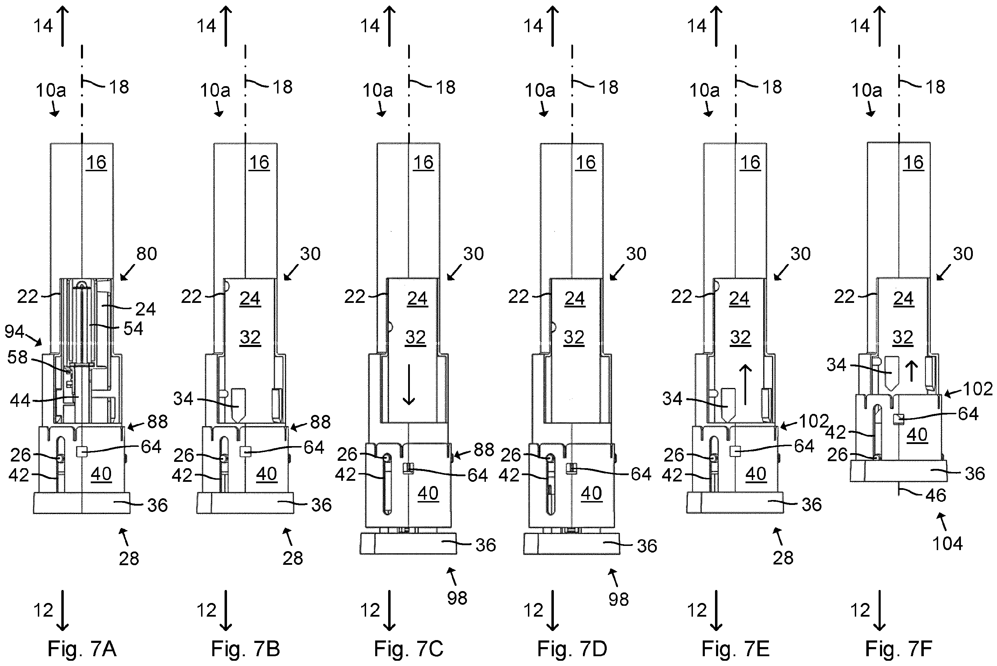

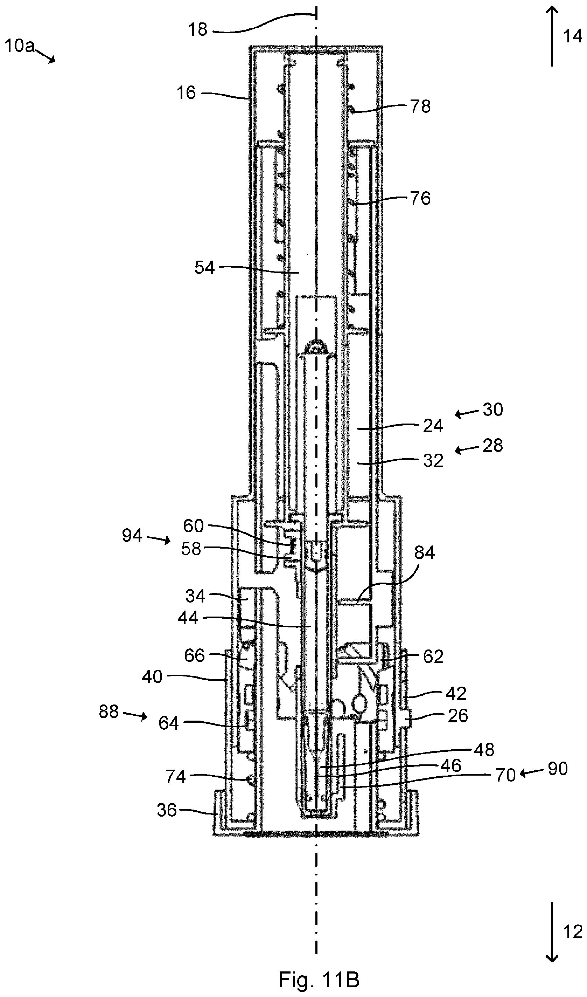

Fig. 11B: schematically represents a cross-sectional side view of the mechanism according to Figs. 7B, 8B and 9B;

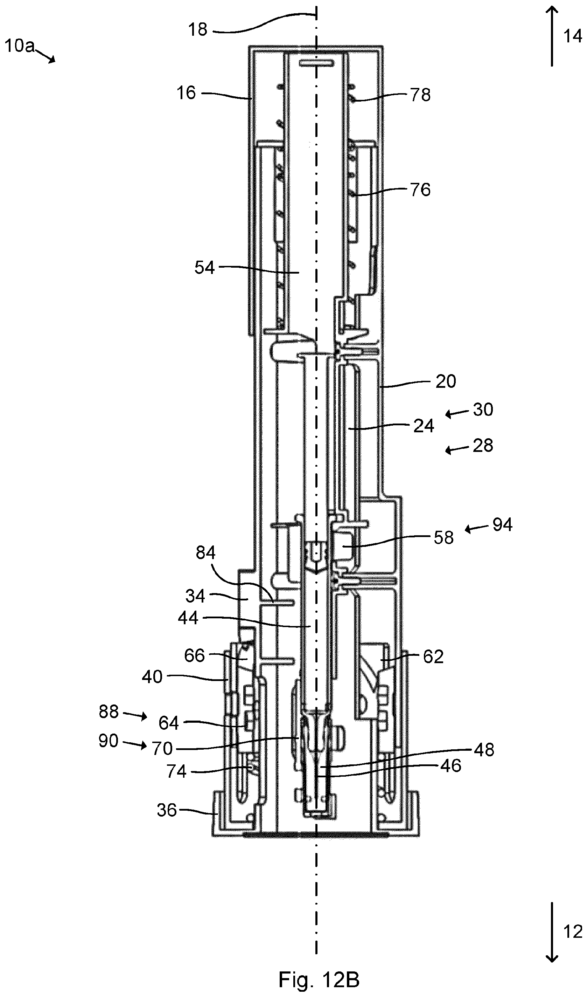

Fig. 12B: schematically represents a further cross-sectional side view of the mechanism according to Figs. 7B, 8B, 9B and 11B;

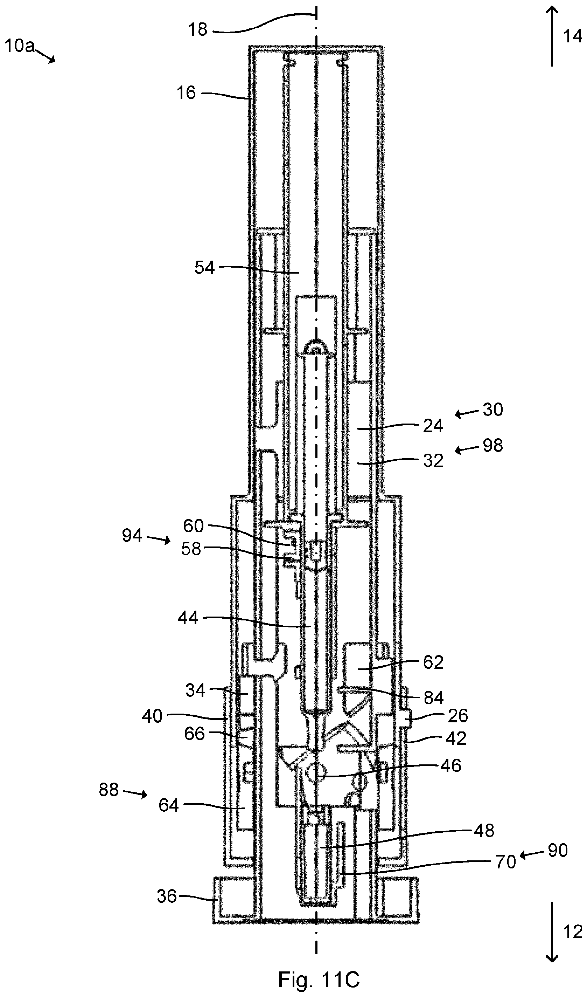

Fig. 11C: schematically represents a cross-sectional side view of the mechanism according to Figs. 7C, 8C and 9C;

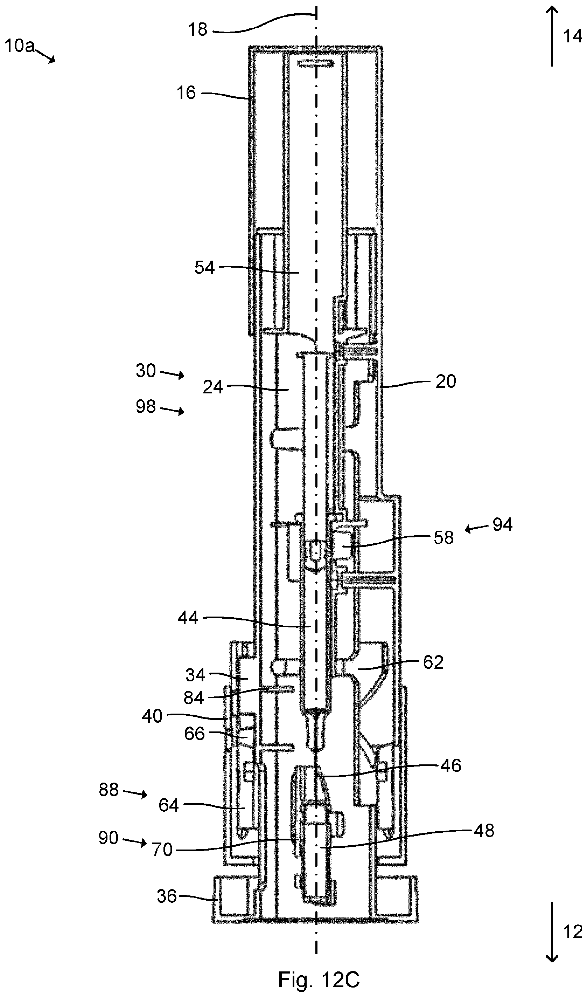

Fig. 12C: schematically represents a further cross-sectional side view of the mechanism according to Figs. 7C, 8C, 9C and 11C;

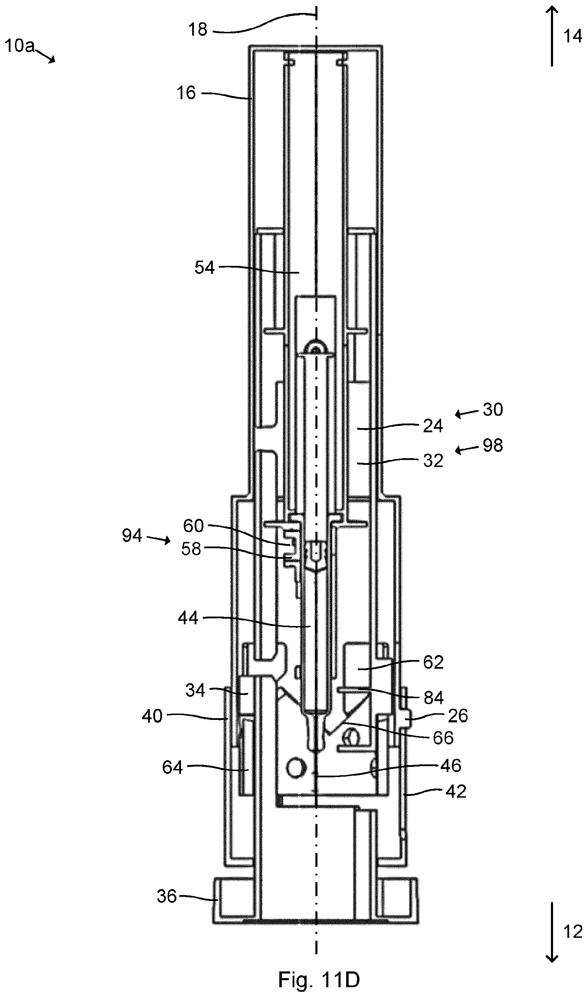

Fig. 11D: schematically represents a cross-sectional side view of the mechanism according to Figs. 7D, 8D and 9D;

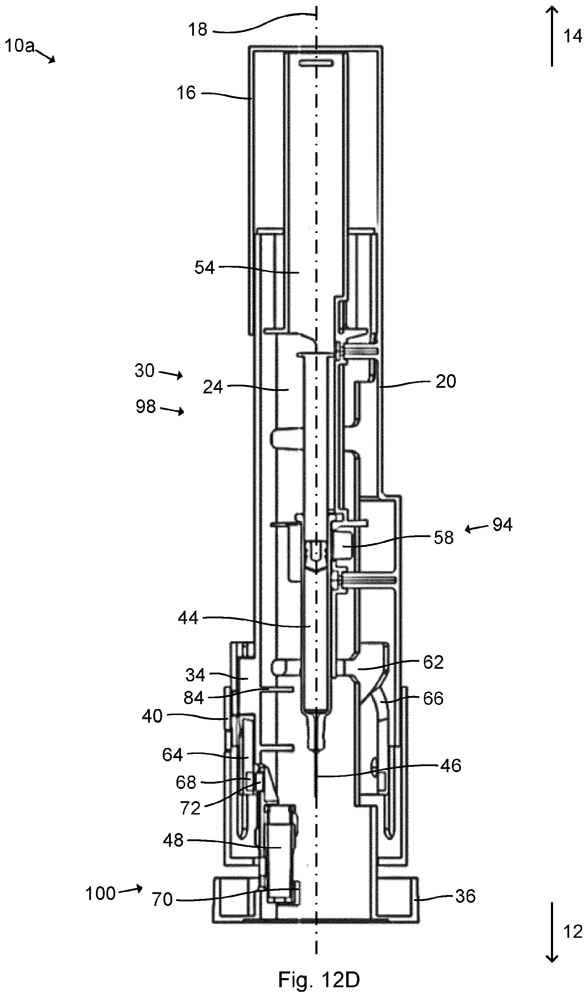

Fig. 12D: schematically represents a further cross-sectional side view of the mechanism according to Figs. 7D, 8D, 9D and 11D;

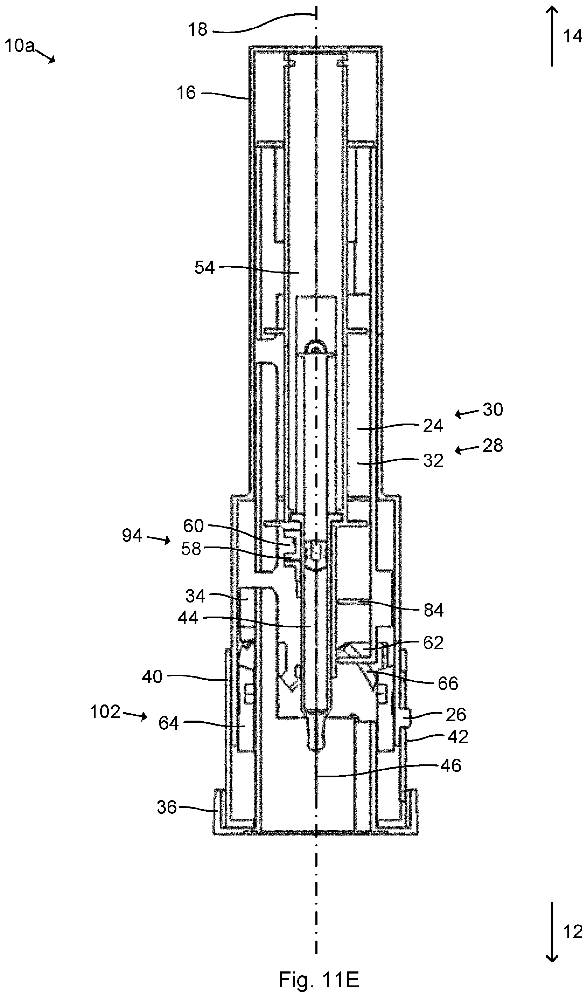

Fig. 11E: schematically represents a cross-sectional side view of the mechanism according to Figs. 7E, 8E and 9E;

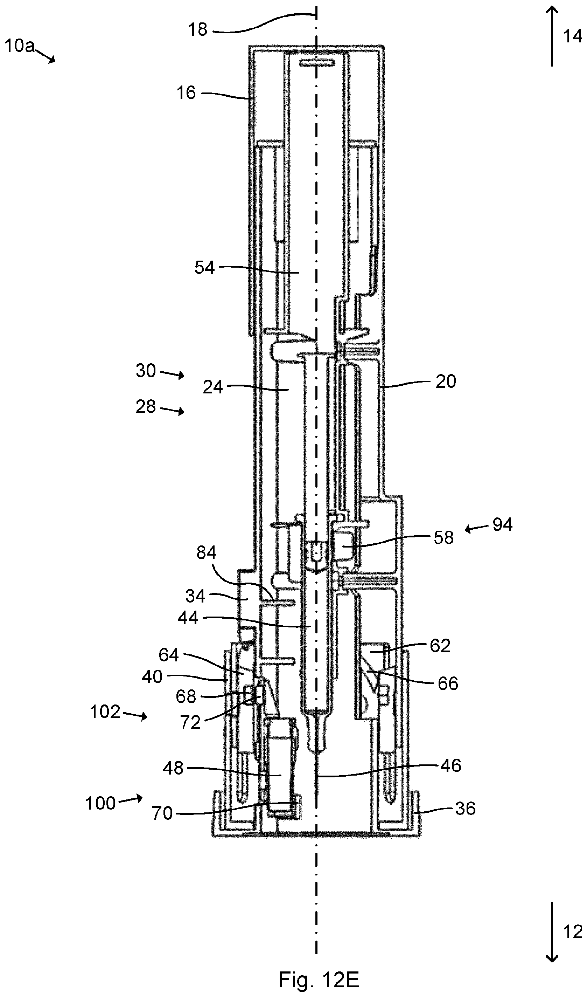

Fig. 12E: schematically represents a further cross-sectional side view of the mechanism according to Figs. 7E, 8E, 9E and 11E;

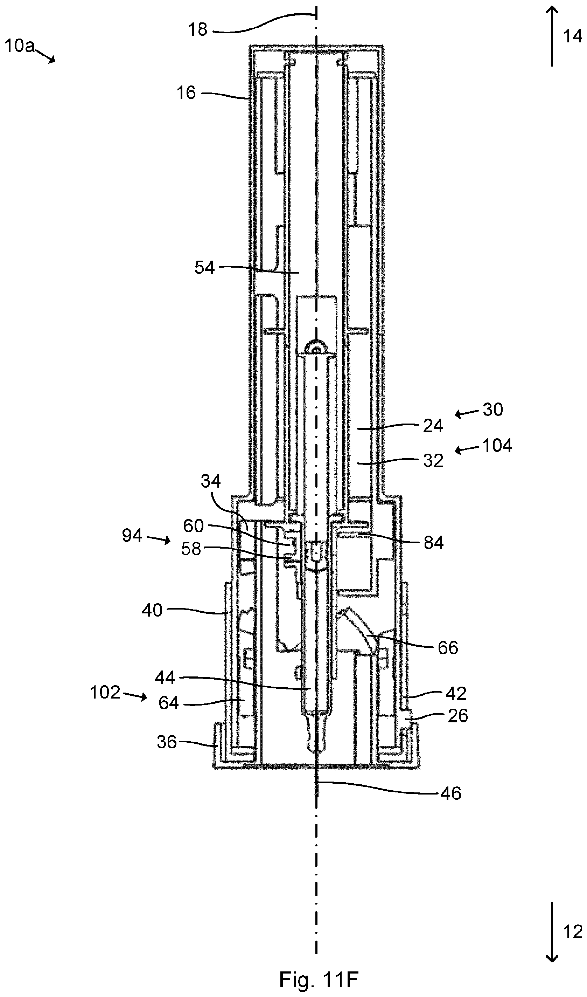

Fig. 11F: schematically represents a cross-sectional side view of the mechanism according to Figs. 7F, 8F and 9F;

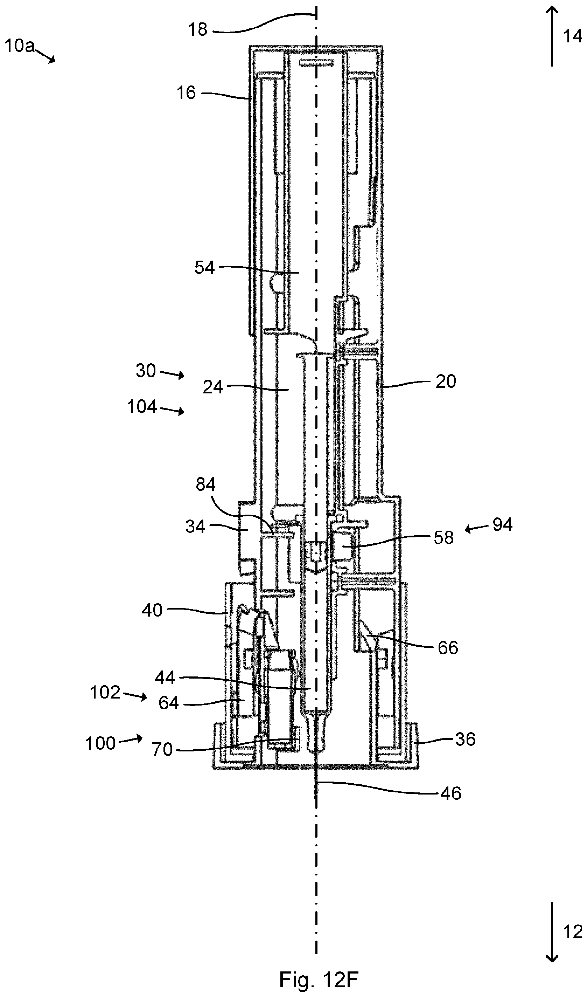

Fig. 12F: schematically represents a further cross-sectional side view of the mechanism according to Figs. 7F, 8F, 9F and 11F;

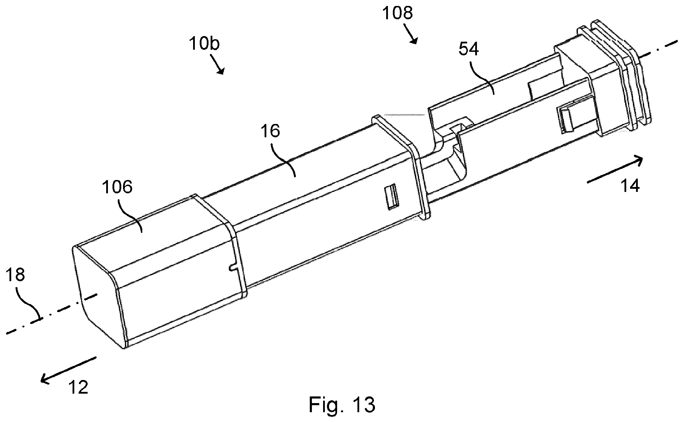

Fig. 13: schematically represents a perspective view of a further mechanism where an injector holder is in a loading position;

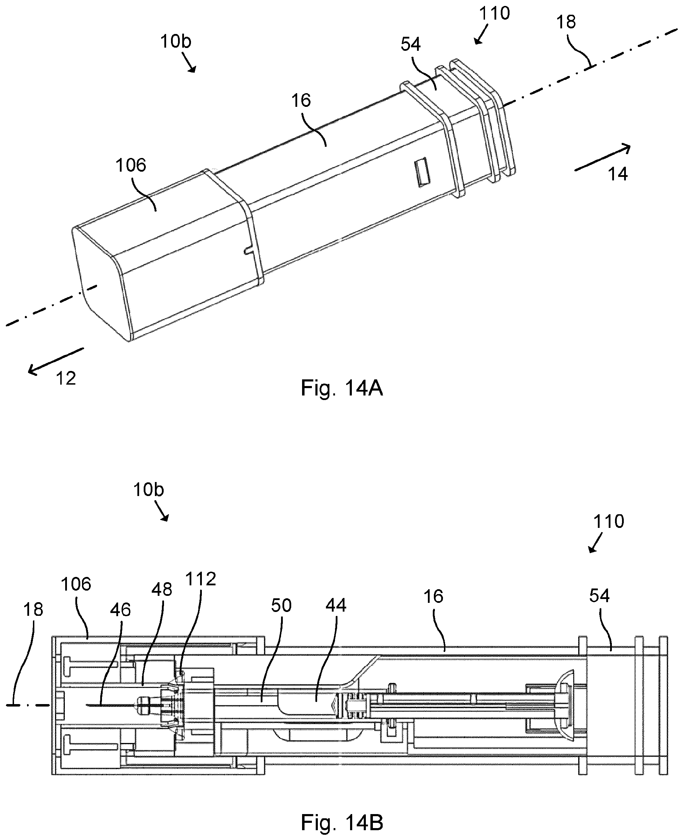

Fig. 14A: schematically represents a perspective view of the mechanism in Fig. 13 where the injector holder is in a delivery position;
Fig. 14B: schematically represents a cross-sectional side view of the mechanism in Fig. 14A;

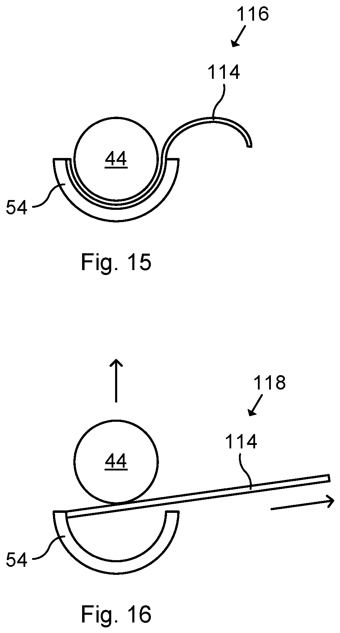

Fig. 15: schematically represents a partial proximal view of a strap in a relaxed state;
Fig. 16: schematically represents a partial proximal view of the strap in a tensioned state;

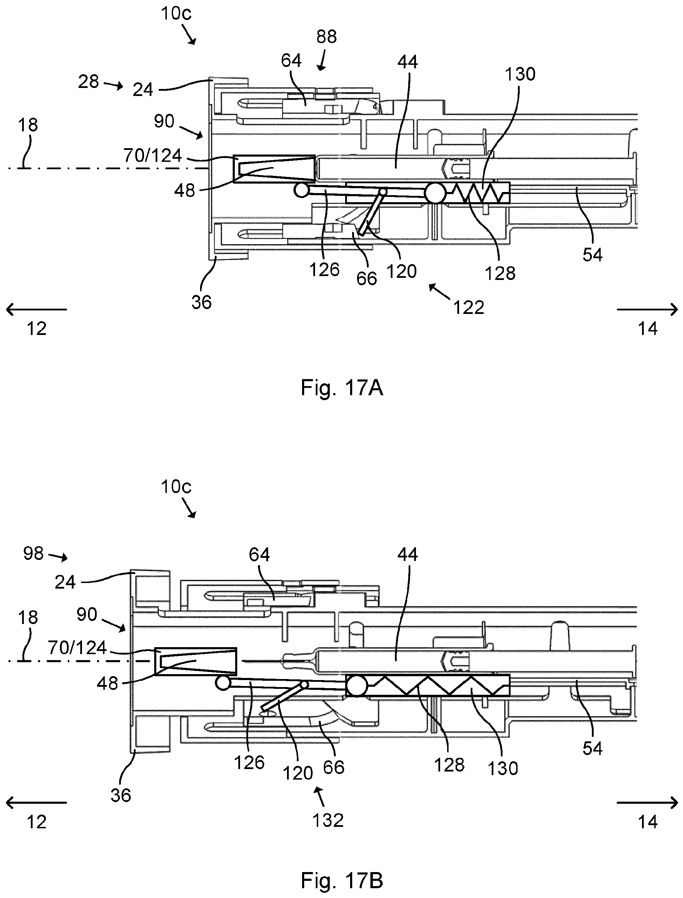

Fig. 17A: schematically represents a partial cross-sectional side view of a further mechanism where a stop arm is in a stopping position and the actuating body is in the first actuating position;
Fig. 17B: schematically represents a partial cross-sectional side view of the mechanism in Fig. 17A when the stop arm is in a releasing position and the actuating body is in the second actuating position;

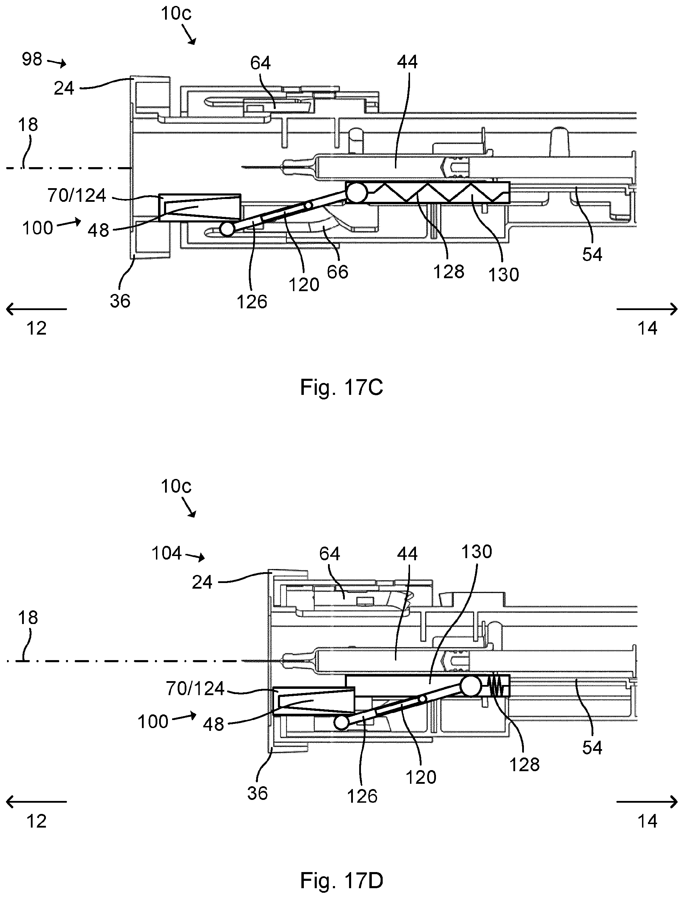

Fig. 17C: schematically represents a partial cross-sectional side view of the mechanism in Figs. 17A and 17B when a needle shield is in an offset position; and
Fig. 17D: schematically represents a partial cross-sectional side view of the mechanism in Figs. 17A to 17C when the actuating body is in the third actuating position.

### Detailed Description

In the following, a mechanism for receiving an injector having a needle and a needle shield covering the needle, where the mechanism comprises a shield holder movable between an aligned position and an offset position, and a mechanism for receiving an injector having a needle and a needle shield covering the needle, where the mechanism comprises an injector holder movable from a loading position to a delivery position, will be described. The same or similar reference numerals will be used to denote the same or similar structural features.

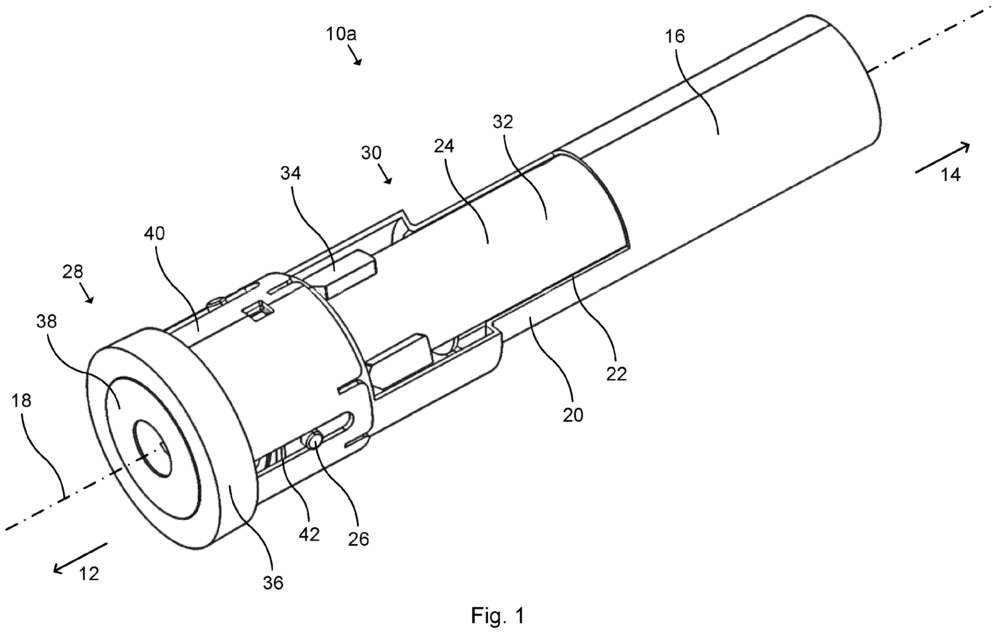

Fig. 1 schematically represents a perspective view of a mechanism 10a for receiving an injector. The mechanism 10a is here exemplified as a reusable autoinjector. An injector can be loaded into the mechanism 10a and the mechanism 10a can then be used to deliver medicament from the injector to an injection site. Fig. 1 also shows a proximal direction 12 and a distal direction 14.

The mechanism 10a comprises a housing 16 and a longitudinal axis 18. The housing 16 of this example is generally cylindrical and centered with respect to the longitudinal axis 18. The housing 16 comprises a housing wall 20. The housing 16 further comprises an opening 22 in the housing wall 20.

The housing 16 of this example further comprises an actuating body 24. Also the actuating body 24 is cylindrical and centered with respect to the longitudinal axis 18. In this example, the actuating body 24 is arranged inside the housing 16.

The housing 16 further comprises a plurality of housing pins 26. Each housing pin 26 extends radially outwards with respect to the longitudinal axis 18 from the housing 16. In this example, the housing 16 comprises three housing pins 26.

The actuating body 24 is movable relative to the housing 16 along the longitudinal axis 18, as described below. In Fig. 1, the actuating body 24 is in a first actuating position 28.

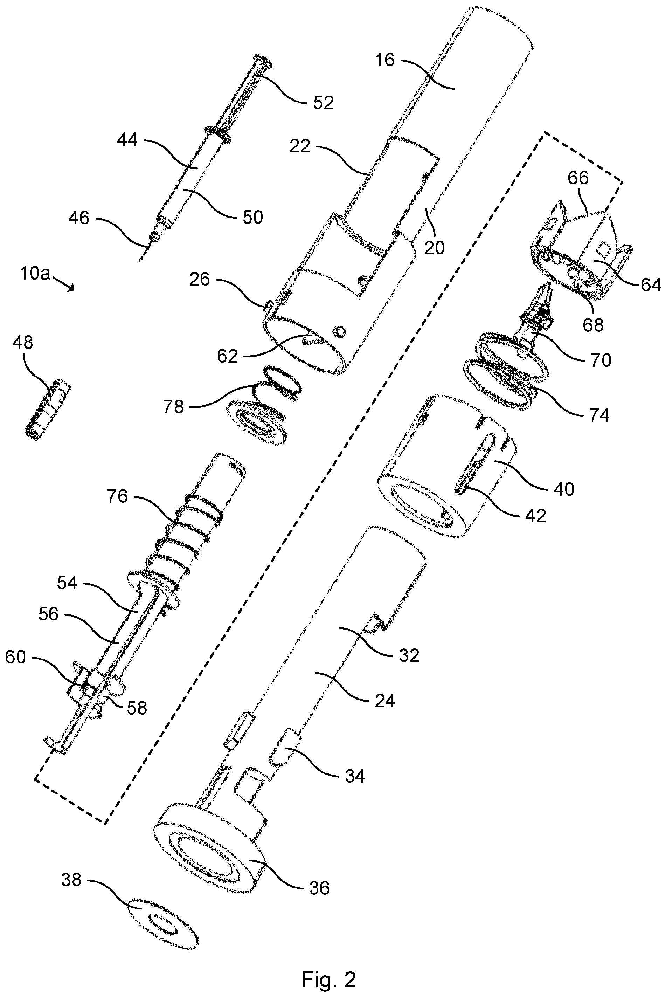

The actuating body 24 is also rotatable relative to the housing 16 about the longitudinal axis 18 between an open body position and a closed body position 30. The actuating body can be assembled by multiple pieces or be formed as one single piece component through injection molding, as shown in Fig. 2. In Fig. 1, the actuating body 24 is in the closed body position 30. In the closed body position 30, the housing 16 and the actuating body 24 together enclose the injector inside the mechanism 10a.

The actuating body 24 comprises a body wall 32. In the closed body position 30, the body wall 32 completely covers the opening 22 from the inside.

The actuating body 24 further comprises a plurality of cam followers 34. In this example, the actuating body 24 comprises three cam followers 34 (only two are visible in Fig. 1). The cam followers 34 are here arranged on the outside of the body wall 32.

The mechanism 10a further comprises a needle guard 36. The needle guard 36 is here constituted by a proximal part of the actuating body 24. The needle guard 36 has a larger radial extension, than the rest of parts of the actuating body 24, with respect to the longitudinal axis 18 than the body wall 32.

The mechanism 10a of this example further comprises a cover plate 38. The cover plate 38 functions to hide the components inside the mechanism 10a.

The mechanism 10a of this example further comprises a sleeve 40. The sleeve 40 is one example of a guiding member according to the present disclosure. The sleeve 40 is arranged to limit movements of the actuating body 24 relative to the housing 16 along the longitudinal axis 18. The sleeve 40 is centered with respect to the longitudinal axis 18.

The sleeve 40 comprises a plurality of guiding grooves 42, each for receiving one of the housing pins 26. Each guiding groove 42 extends in parallel with the longitudinal axis 18. The guiding grooves 42 are evenly distributed around the circumference of the sleeve 40. By means of the housing pins 26 engaging in the guiding grooves 42, the sleeve 40 is arranged to move relative to the housing 16 along the longitudinal axis 18, and is prevented from rotating relative to the housing 16 about the longitudinal axis 18.

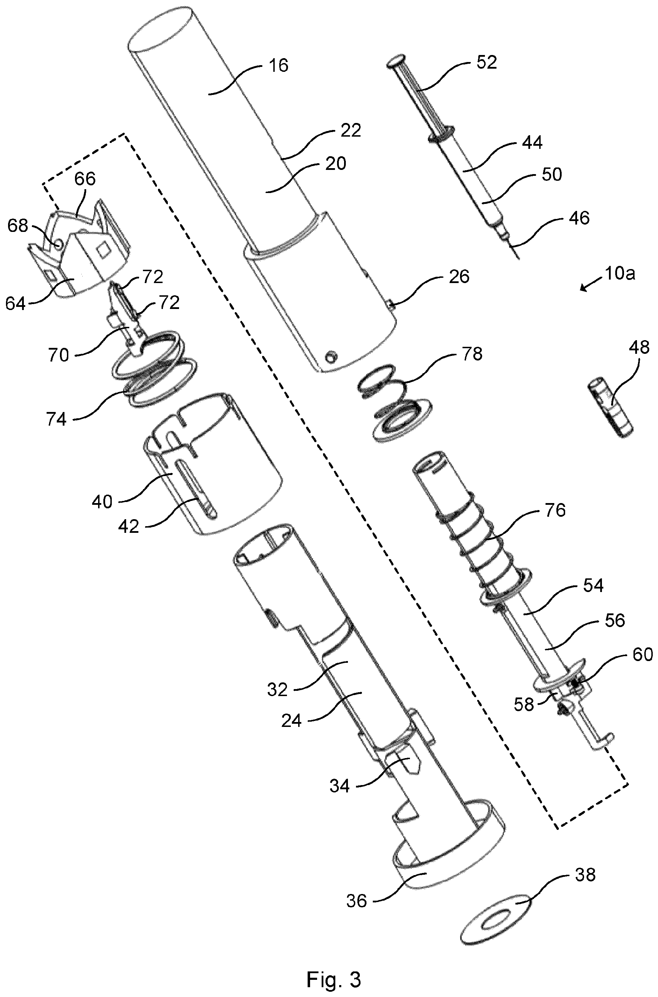

Fig. 2 schematically represents a perspective exploded view of the mechanism 10a and the injector 44, and Fig. 3 schematically represents a further perspective exploded view of the mechanism 10a and the injector 44. With collective reference to Figs. 2 and 3, the injector 44 comprises a needle 46 and a needle shield 48 for covering the needle 46. The injector 44 of this example is a syringe. The injector 44 further comprises a medicament container 50 with medicament, and a plunger 52.

The mechanism 10a further comprises an injector holder 54. The injector holder 54 may be integrally formed with, or fixedly attached to, the housing 16. The injector holder 54 thus forms a part of the housing 16. As its name implies, the injector holder 54 is arranged to hold the injector 44. The injector holder 54 is arranged to hold the injector 44 such that the injector 44 is oriented coaxially with the longitudinal axis 18. The injector holder 54 is here exemplified as an injector seat.

The injector holder 54 of this example comprises a support body 56 and a lifter 58. The lifter 58 is movable relative to the support body 56 between a lowered position and a raised position. In this example, the lifter 58 is rotatable relative to the support body 56 about a lifter axis, parallel with the longitudinal axis 18.

The injector holder 54 of this example further comprises a lifter spring 60. The lifter spring 60 is one example of a lifter force device according to the present disclosure. The lifter spring 60 is here a torsion spring arranged around the lifter axis.

The housing 16 further comprises a cam follower 62 (Fig. 2). The cam follower 62 extends radially inwards with respect to the longitudinal axis 18 from the housing 16.

The mechanism 10a further comprises a rotator 64. The rotator 64 is rotatable relative to the housing 16 about the longitudinal axis 18, as described below. In the presented example, the rotator 64 is a tubular shape component; however, the rotator could be formed as a semi-circular or other suitable shaped component. In this example, the actuating body 24 is partially enclosed by the rotator 64 and the rotator 64 is arranged inside the housing 16.

The rotator 64 comprises a cam profile 66. The cam profile 66 is here provided on a distal end of the rotator 64. The cam profile 66 of this example has a zig-zag shape forming four V-shaped peaks and four V-shaped valleys alternatingly arranged around the circumference of the rotator 64. The four peaks point in distal direction 14 and the four valleys point in the proximal direction 12.

The rotator 64 further comprises a plurality of magnets 68. The magnets 68 are here arranged on the interior surface of the rotator 64 to face towards the longitudinal axis 18. The magnets 68 are spaced in the circumferential direction of the rotator 64 around the longitudinal axis 18. The rotator 64 of this example comprises six magnets 68. Alternatively, the rotator 64 may comprise more than six magnets. Some of the magnets 68 have a pole of a first polarity facing towards the longitudinal axis 18, and some of the magnets 68 have a pole of a second polarity, different from the first polarity, facing towards the longitudinal axis 18. For example, four magnets 68 may have a south pole facing towards the longitudinal axis 18 and two magnets 68 may have a north pole facing towards the longitudinal axis 18.

The mechanism 10a further comprises a shield holder 70, here exemplified as a shield seat. As its name implies, the shield holder 70 is configured to hold the needle shield 48. The shield holder 70 is arranged radially inside the housing 16 with respect to the longitudinal axis 18.

The shield holder 70 comprises one or more magnets 72. In this example, the shield holder 70 comprises two magnets 72. The magnets 72 are laterally arranged on the shield holder 70 or arranged on a common side of the shield holder 70. When the shield holder 70 is aligned with the longitudinal axis 18, the magnets 72 face radially outwards with respect to the longitudinal axis 18. The magnets 72 on the shield holder 70 cooperate with the magnets 68 on the rotator 64, as will be described below.

The mechanism 10a further comprises a rotator spring 74. The rotator spring 74 is one example of a rotator force device according to the present disclosure. The rotator spring 74 is here exemplified as a compression coil spring. The rotator spring 74 is arranged to force the rotator 64 along the longitudinal axis 18 in the distal direction 14. In this example, a proximal end of the rotator spring 74 is in contact with the sleeve 40 and a distal end of the rotator spring 74 is in contact with the rotator 64.

The mechanism 10a further comprises a primary spring 76 and a secondary spring 78. The primary spring 76 and the secondary spring 78 are examples of a primary force device and a secondary force device, respectively, according to the present disclosure. Each of the primary spring 76 and the secondary spring 78 is here exemplified as a compression coil spring.

The primary spring 76 is arranged to force the actuating body 24 in the distal direction 14. To this end, a proximal end of the primary spring 76 is in contact with the injector holder 54 and a distal end of the primary spring 76 is in contact with the actuating body 24.

The secondary spring 78 is arranged to force the actuating body 24 in the proximal direction 12. To this end, a proximal end of the secondary spring 78 is in contact with the actuating body 24 and a distal end of the secondary spring 78 is in contact with the housing 16.

In this example, the rotator spring 74, the primary spring 76 and the secondary spring 78 provide the driving power for injection and for removing the needle shield 48 by means of the shield holder 70, as described below.

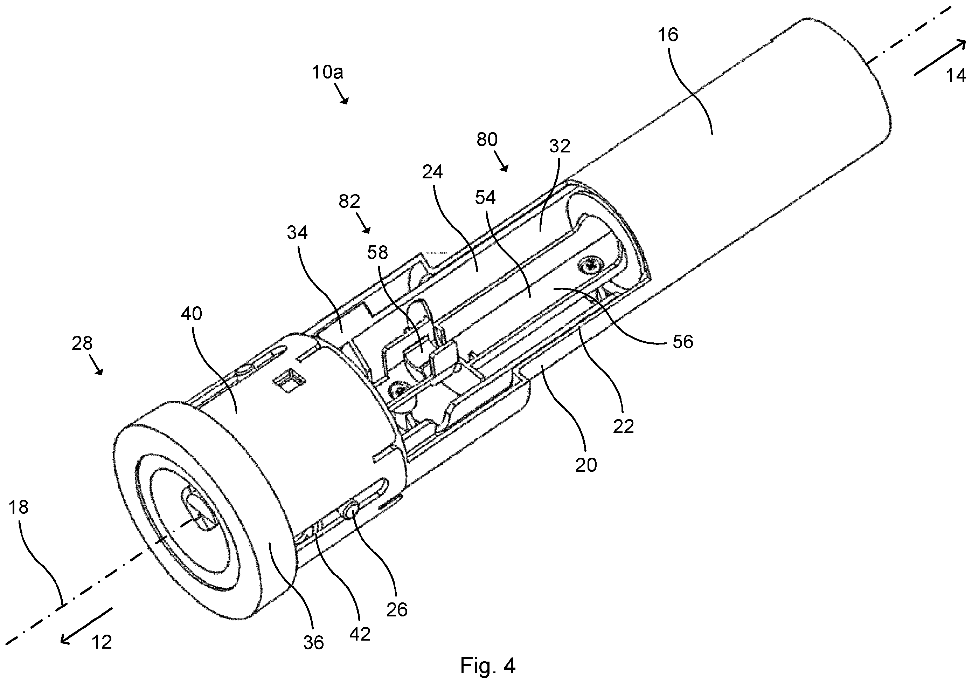

Fig. 4 schematically represents a perspective view of the mechanism 10a when an actuating body 24 is in the open body position 80. In order to open the mechanism 10a to the state shown in Fig. 4, the user may grab the needle guard 36 with one hand, grab the housing 16 with the other hand, and rotate the actuating body 24 from the closed body position 30 to the open body position 80, and vice versa. In the open body position 80 of the actuating body 24, the injector 44 can be loaded into the mechanism 10a such that the injector 44 is received by the injector holder 54.

In the open body position 80, the housing wall 20 overlaps the body wall 32 of the actuating body 24. Moreover, the body wall 32 is positioned between the housing wall 20 and the injector holder 54 in a radial direction with respect to the longitudinal axis 18 when the actuating body 24 is in the open body position 80.

Fig. 4 further shows that the lifter 58 is in a raised position 82. In the raised position 82, the lifter 58 is raised relative to the support body 56, namely, the lifter 58 is moved at least partially radially relative to the longitudinal axis 18 and toward the opening 22. In this example, the lifter spring 60 forces the lifter 58 to rotate about the lifter axis into the raised position 82.

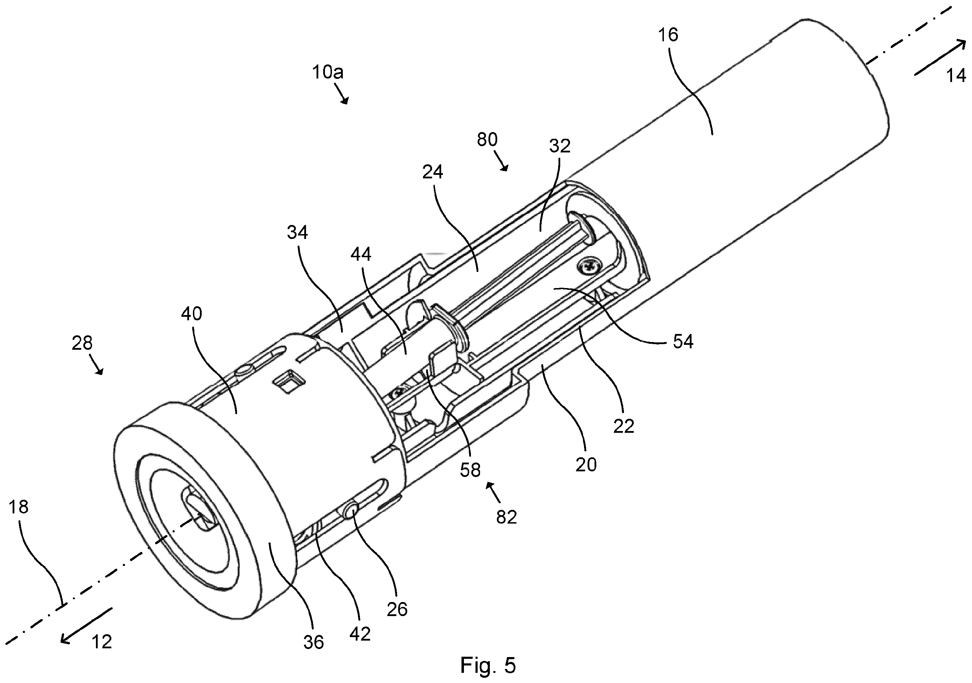

Fig. 5 schematically represents a perspective view of the mechanism 10a when receiving the injector 44. When the lifter 58 is in the raised position 82, the lifter 58 holds the injector 44 at an angle to the longitudinal axis 18. The injector 44 is thereby raised from the injector holder 54. In the raised position 82 of the lifter 58, the injector 44 can be easily accessed for grabbing by a user.

After the user has put the injector 44 into the mechanism 10a, the user can now twist the mechanism 10a in an opposite direction by one hand grabbing the needle guard 36 and by one hand grabbing the housing 16 to effect rotation of the actuating body 24 from the open body position 80 to the closed body position 30. This rotation of the actuating body 24 causes the lifter 58 to move from the raised position 82 to the lowered position and the injector 44 to be arranged concentric with the longitudinal axis 18, as described below.

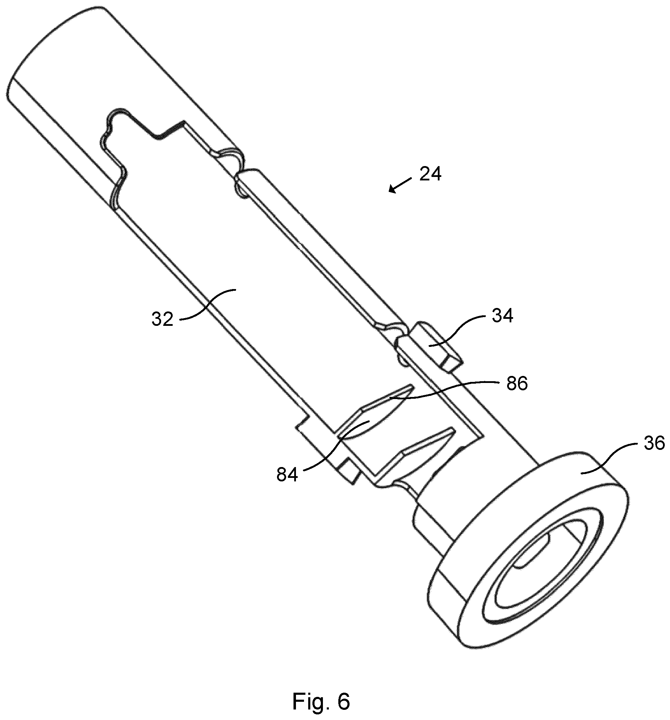

Fig. 6 schematically represents a perspective view of the actuating body 24 of the mechanism 10a. As shown, the actuating body 24 comprises a pushing structure 84. The pushing structure 84 is here exemplified as two walls, each having an edge 86. Alternatively, the pushing structure can be formed by more than two walls. In presented example, each wall of the pushing structure 84 extends in a direction perpendicular to the longitudinal axis 18. Each edge 86 is perpendicular to, and offset from, the longitudinal axis 18.

The pushing structure 84 is arranged to push the injector 44 into alignment with the longitudinal axis 18 when the actuating body 24 is rotated from the open body position 80 to the closed body position 30. This movement of the injector 44 in turn pushes the lifter 58 to be moved from the raised position 82 to the lowered position against the force of the lifter spring 60.

Fig. 7A-7F illustrate the side views of the mechanism from the outside of the housing 16. It should be noted that, the rotator 64 is mainly blocked from viewing by the housing 16 in Fig. 7A-7F; the reference numbers and the pointing lines for the rotator 64 in Fig. 7A-7F are pointing to the rotator through a window on the sleeve 40. The window is configured to remind the user of the current status of the mechanism. For example, the rotator may comprise few different colors on the outer surface, such as red and green color. Those colors are configured to align with the window, so that the user is able to observe from outside and understand what rotation position that the rotator is located. The rotation position of the rotator can be an indication of the status of the mechanism (will be example in detail below). The user can observe the window, and see, for example, a green color indicating that the user can start the injection process; or the red color indicating that the user should change a new injector (indicating an accomplishment of an injection).

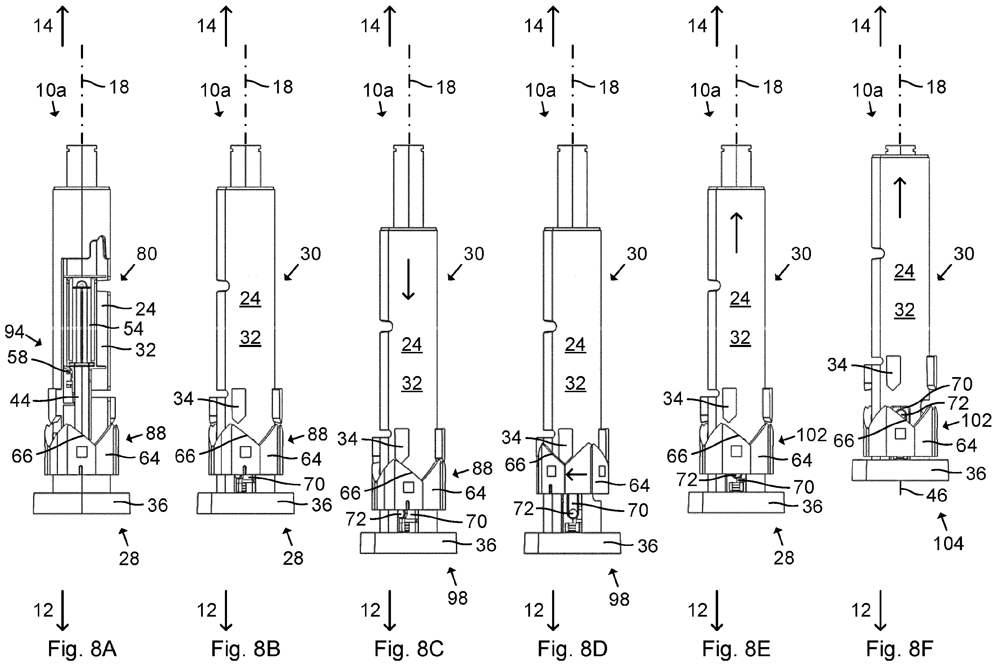

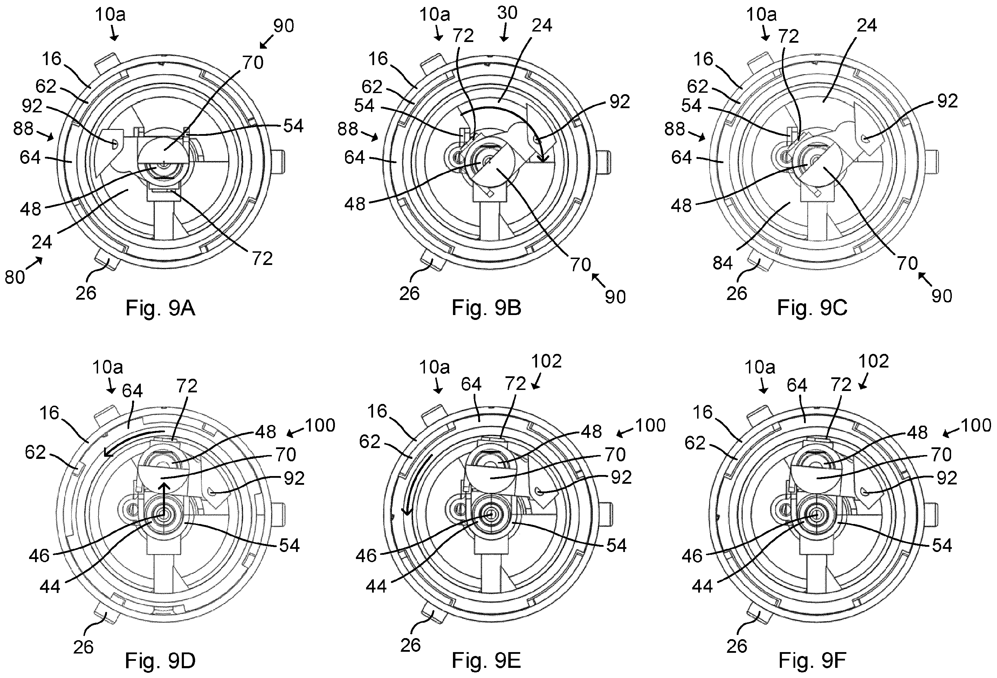

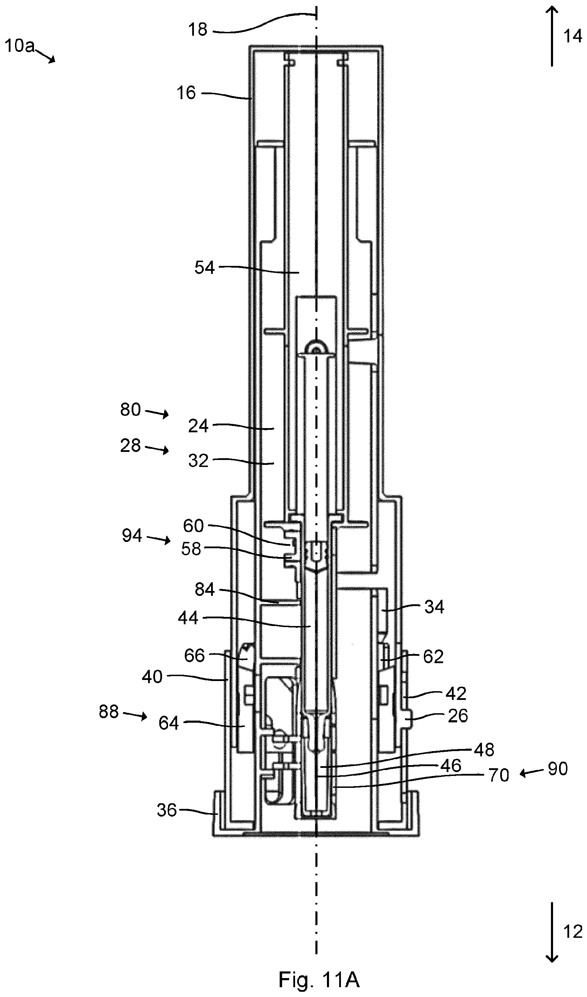

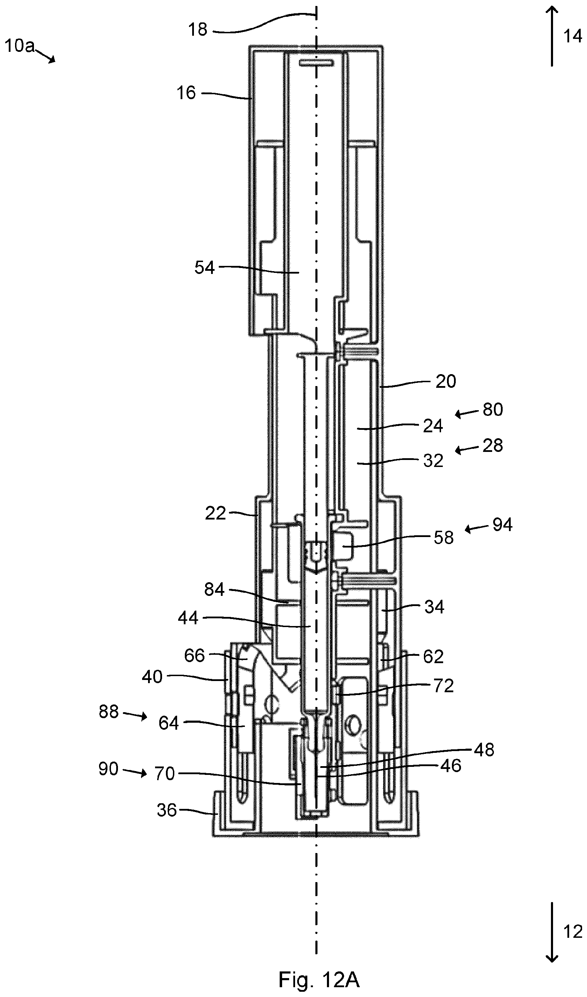

Fig. 7A schematically represents a side view of the mechanism 10a when the actuating body 24 is in the open body position 80 and in the first actuating position 28. Fig. 8A schematically represents a partial side view of the mechanism 10a according to Fig. 7A, Fig. 9A schematically represents a partial distal view of the mechanism 10a according to Fig. 7A, Fig. 11A schematically represents a cross-sectional side view of the mechanism 10a according to Fig. 7A, and Fig. 12A schematically represents a further cross-sectional side view of the mechanism 10a according to Fig. 7A. With collective reference to Figs. 7A, 8A, 9A, 11A and 12A, the mechanism 10a is in an initial position. The user has put the injector 44 into the mechanism 10a (see also Fig. 5). When the injector 44 is inserted into the mechanism 10a, the needle shield 48 is received in the shield holder 70. In the initial position, the rotator 64 is in a first rotator position 88 with respect to the longitudinal axis 18. In the first actuating position 28, the needle guard 36 of the actuating body 24 covers the needle 46.

As shown in Fig. 9A, the shield holder 70 is in an aligned position 90. In the aligned position 90, the shield holder 70 holds the needle shield 48 aligned with the longitudinal axis 18. In this example, the shield holder 70 is rotatably connected to the actuating body 24 about a shield holder pivot 92. The shield holder pivot 92 is parallel with, and offset from, the longitudinal axis 18.

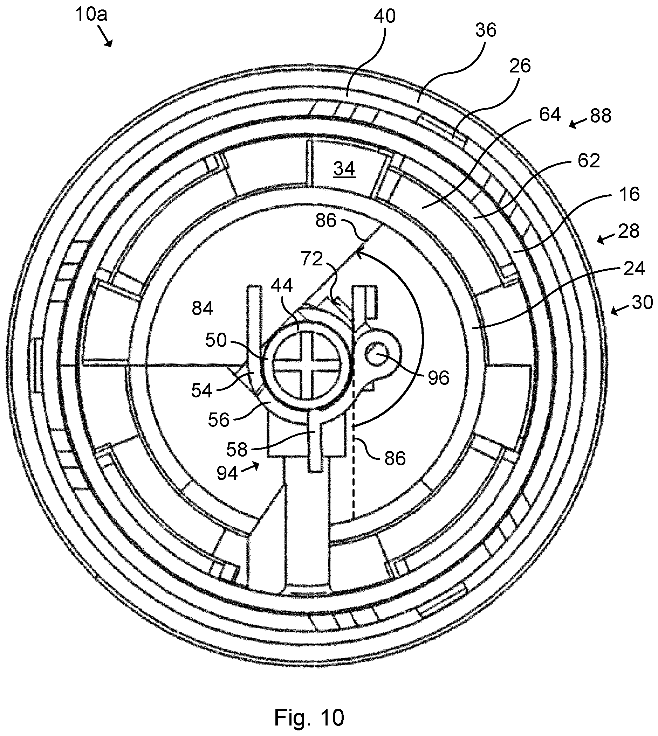

Fig. 7B schematically represents a side view of the mechanism 10a when the actuating body 24 is in the closed body position 30. Fig. 8B schematically represents a partial side view of the mechanism 10a according to Fig. 7B, Fig. 9B schematically represents a partial distal view of the mechanism 10a according to Fig. 7B, Fig. 10 schematically represents a cross-sectional proximal view of the mechanism 10a in Fig. 7B, Fig. 11B schematically represents a cross-sectional side view of the mechanism 10a according to Fig. 7B, and Fig. 12B schematically represents a further cross-sectional side view of the mechanism 10a according to Fig. 7B. With collective reference to Figs. 7B, 8B, 9B, 10, 11B and 12B, the user rotates the actuating body 24 120 degrees (clockwise in Fig. 9B) from the open body position 80 to the closed body position 30. The mechanism 10a is thereby closed and locked. The shield holder 70 rotates together with the actuating body 24.

During rotation of the actuating body 24 from the open body position 80 to the closed body position 30, the cam followers 34 on the actuating body 24 are positioned distally of the rotator 64 and do therefore not interact with the cam profile 66.

With particular reference to Fig. 10, during rotation of the actuating body 24 from the open body position 80 to the closed body position 30, the pushing structure 84 rotates about the longitudinal axis 18. During this rotation of the actuating body 24, the injector 44 and the lifter 58 are at least partially moved radially relative to the longitudinal axis 18 and therefore moved away the opening 22, and moved toward the longitudinal axis 18, namely the lifter 58 and the injector 44 is pushed down. The pushing structure 84 pushes on the injector 44 which in turn pushes on the lifter 58. This causes the lifter 58 to be pushed down from the raised position 82 to the lowered position 94. In the lowered position 94 of the lifter 58, the injector 44 is seated in the injector holder 54 in an orientation concentric with the longitudinal axis 18.

The position of the edge 86 when the actuating body 24 is in the open body position 80 is shown with a dashed line in Fig. 10. When the edge 86 is rotated in the counterclockwise direction as seen in Fig. 10 as the actuating body 24 moves from the open body position 80 to the closed body position 30, the edge 86 gradually pushes the injector 44 into the injector holder 54. The lifter 58 is thereby caused to rotate (counterclockwise in Fig. 10) about the lifter axis 96 from the raised position 82 to the lowered position 94. In this way, the mechanism 10a is arranged to transmit a movement of the actuating body 24 from the open body position 80 to the closed body position 30 to a movement of the lifter 58 from the raised position 82 to the lowered position 94.

As further shown in Fig. 10, when the actuating body 24 is in the closed body position 30, the edge 86 securely holds the injector 44 in the injector holder 54 and thereby holds the lifter 58 in the lowered position 94. In the closed body position 30 of the actuating body 24, the edge 86 is substantially perpendicular to a contact force exerted by the injector 44 on the edge 86 by means of the force from the lifter spring 60.

Fig. 7C schematically represents a side view of the mechanism 10a. In Fig. 7C, the actuating body 24 has moved in the proximal direction 12 from the first actuating position 28 to a second actuating position 98. This movement can be accomplished by the user by grabbing the housing 16 with one hand, grabbing the needle guard 36 with the other hand, and pulling these components away from each other. Fig. 8C schematically represents a partial side view of the mechanism 10a according to Fig. 7C, Fig. 9C schematically represents a partial distal view of the mechanism 10a according to Fig. 7C, Fig. 11C schematically represents a cross-sectional side view of the mechanism 10a according to Fig. 7C, and Fig. 12C schematically represents a further cross-sectional side view of the mechanism 10a according to Fig. 7C. With collective reference to Figs. 7C, 8C, 9C, 11C and 12C, the movement of the actuating body 24 from the first actuating position 28 to the second actuating position 98 compresses the primary spring 76. Also in the second actuating position 98, the needle guard 36 of the actuating body 24 covers the needle 46.

As the actuating body 24 is moved in the proximal direction 12, the rotator spring 74 pushes the sleeve 40 in the proximal direction 12. At the same time, or at substantially the same time, the cam followers 34 are brought into contact with the cam profile 66 (Fig. 8C). The sleeve 40 travels in the proximal direction 12 until the housing pins 26 reach a respective distal end of the guiding grooves 42 (Fig. 7C). The sleeve 40 controls the travel of the actuating body 24. The movement of the actuating body 24 in the proximal direction 12 causes compression of the rotator spring 74.

As shown in Figs. 11C and 12C, the shield holder 70 follows the movement of the actuating body 24. The movement of the actuating body 24 from the first actuating position 28 to the second actuating position 98 thus causes also the shield holder 70 to move in the proximal direction 12, since the shield holder 70 and the needle shield 48 is fixedly attached, the movement of the shield holder 70 causes the needle shield 48 to move in the proximal direction 12. The proximal movement of the needle shield 48 causes the needle shield 48 to detach from the needle 46 in the proximal direction 12. However, since the needle shield 48 is fixedly attached to the shield holder 70, when the needle shield 48 is completely detached from the needle 46, the needle shield 48 will still be fixed to the shield holder 70, and therefore the needle shield 48 will still be received within the housing 16. The shield holder 70 however remains in the aligned position 90 as long as the rotator 64 is in the first rotator position 88.

Fig. 7D schematically represents a side view of the mechanism 10a when the rotator 64 has rotated from the first rotator position 88. Fig. 8D schematically represents a partial side view of the mechanism 10a according to Fig. 7D, Fig. 9D schematically represents a partial distal view of the mechanism 10a according to Fig. 7D, Fig. 11D schematically represents a cross-sectional side view of the mechanism 10a according to Fig. 7D, and Fig. 12D schematically represents a further cross-sectional side view of the mechanism 10a according to Fig. 7D. The rotator spring 74 forces the rotator 64 in the distal direction 14. This force together with the engagement between the cam followers 34 and the cam profile 66 cause the rotator 64 to rotate (Figs. 8D and 9D). In this example, the rotator 64 is rotated 45 degrees around the longitudinal axis 18 (counterclockwise in Fig. 9D) from the first rotator position 88 until each cam follower 34 is seated in an associated valley of the cam profile 66.

During the rotation of the rotator 64 about the longitudinal axis 18, one or more magnets 68 on the rotator 64 are brought into alignment with the magnets 72 on the shield holder 70. The shield holder 70 is thereby forced to move from the aligned position 90 to an offset position 100 by means of magnetic attraction (Fig. 9D). In the offset position 100, the shield holder 70 and the needle shield 48 are positioned aside the injector 44 or positioned radially with respect to the longitudinal axis 18 relative to the injector 44. The shield holder 70 and the needle shield 48 do thereby not obstruct the needle 46. In this example, the shield holder 70 is almost touching the interior of the rotator 64 when the shield holder 70 is in the offset position 100. The shield holder 70 moves relative to the housing 16 between the aligned position 90 and the offset position 100.

Fig. 7E schematically represents a side view of the mechanism 10a when the actuating body 24 is in the first actuating position 28 and the rotator 64 is in a second rotator position 102. Fig. 8E schematically represents a partial side view of the mechanism 10a according to Fig. 7E, Fig. 9E schematically represents a partial distal view of the mechanism 10a according to Fig. 7E, Fig. 11E schematically represents a cross-sectional side view of the mechanism 10a according to Fig. 7E, and Fig. 12E schematically represents a further cross-sectional side view of the mechanism 10a according to Fig. 7E. With collective reference to Figs. 7E, 8E, 9E, 11E and 12E, when the manual pulling force has been released, the mechanism 10a is now ready for injection.

When the user releases the grip of the actuating body 24, the primary spring 76 forces the actuating body 24 in the distal direction 14 from the second actuating position 98 back to the first actuating position 28. During this movement, the cam followers 62 on the housing 16 engage the cam profile 66 on the rotator 64. This engagement causes the rotator 64 to be rotated further. The rotator 64 is in this example now rotated additionally 45 degrees around the longitudinal axis 18 (counterclockwise in Fig. 9E). The rotator 64 of this example is thus arranged to rotate from the first rotator position 88 to the second rotator position 102 in response to a proximal movement of the actuating body 24 from the first actuating position 28 to the second actuating position 98, and a subsequent distal movement of the actuating body 24 from the second actuating position 98 back to the first actuating position 28. The rotator 64 of this example rotates 90 degrees about the longitudinal axis 18 from the first rotator position 88 to the second rotator position 102. As particularly shown in Fig. 12E, the shield holder 70 and the needle shield 48 are now positioned next to the needle 46.

Although the shield holder 70 in this example is magnetically forced to move from the aligned position 90 to the offset position 100 in the Fig. 9D position of the rotator 64, the shield holder 70 can alternatively be magnetically forced to move from the aligned position 90 to the offset position 100 in the second rotator position 102 according to Fig. 9E. To this end, the magnets 68 may for example be positioned differently on the rotator 64. In any case, the mechanism 10a is configured to cause a movement of the shield holder 70 from the aligned position 90 to the offset position 100 by means of a movement of the rotator 64 from the first rotator position 88 to the second rotator position 102.

Fig. 7F schematically represents a side view of the mechanism 10a when the actuating body 24 is in a third actuating position 104. Fig. 8F schematically represents a partial side view of the mechanism 10a according to Fig. 7F, Fig. 9F schematically represents a partial distal view of the mechanism 10a according to Fig. 7F, Fig. 11F schematically represents a cross-sectional side view of the mechanism 10a according to Fig. 7F, and Fig. 12F schematically represents a further cross-sectional side view of the mechanism 10a according to Fig. 7F. Figs. 7F, 8F, 9F, 11F and 12F show the mechanism 10a during injection. The user may grab the mechanism 10a, position the needle guard 36 against an injection site and push the mechanism 10a towards the injection site. This causes the actuating body 24 to move in the distal direction 14 from the first actuating position 28 to the third actuating position 104, and the needle 46 to penetrate the injection site. The medicament delivery from the injector 44 to the injection site may be triggered by the distal movement of the needle guard 36 in, as such, known ways. During injection, the shield holder 70 remains in the offset position 100. As shown in Figs. 7A-7F, the first actuating position 28 lies between the more proximal second actuating position 98 and the more distal third actuating position 104. When the mechanism 10a is removed from the injection site, the secondary spring 78 forces the actuating body 24 in the proximal direction 12 from the third actuating position 104 back to the first actuating position 28.

After injection, the above procedure can be substantially reversed to put the needle shield 48 back onto the injector 44 and to open the mechanism 10a for removing the injector 44 from the mechanism 10a. In this reverse procedure, it should however be noted that the rotator 64 rotates in the same direction (in the counterclockwise direction as seen in Figs. 9D and 9E). During this rotation of the rotator 64 about the longitudinal axis 18, one or more further magnets 68 on the rotator 64 are eventually brought into alignment with the magnets 72 on the shield holder 70. The shield holder 70 is thereby forced to move from the offset position 100 back to the aligned position 90 by means of magnetic repulsion. This magnetic repulsion may take place when the rotator 64 has rotated to a third rotator position (not illustrated). The second rotator position 102 lies rotationally between the first rotator position 88 and the third rotator position.

It should be noted that the injector holder 54 and the medicament container 50 of the injector 44 remain stationary inside the housing 16 when the needle shield 48 is removed, during injection, and when the needle shield 48 is put back on the needle 46. The user may take out the used injector 44 after completely the injection, so that the mechanism 10a can then be loaded with a new injector 44 to perform a further medicament delivery procedure.

The needle shield 48 is safely contained inside the mechanism 10a before and after injection by being held by the shield holder 70. There is therefore no risk for the user to lose the needle shield 48, for example by dropping the needle shield 48 on the floor.

Fig. 13 schematically represents a perspective view of a further mechanism 10b. The mechanism 10b is configured to accommodate the injector 44 of the same type as described above. The mechanism 10b in Fig. 13 comprises a housing 16, a cover 106 at a proximal end of the housing 16, and an injector holder 54 movable relative to the housing 16. The cover 106 is detachably connected to the housing 16. In Fig. 13, the injector holder 54 is in a loading position 108. In the loading position 108, the injector 44 can be put into the injector holder 54.

Fig. 14A schematically represents a perspective view of the mechanism 10b in Fig. 13 where the injector holder 54 is in a delivery position 110. Fig. 14B schematically represents a cross-sectional side view of the mechanism 10b in Fig. 14A and additionally with the injector 44 therein. With collective reference to Figs. 14A and 14B, the user can push the injector holder 54 from the loading position 108 to the delivery position 110, and pull out the injector holder 54 from the delivery position 110 to the loading position 108, like a drawer. In the delivery position 110, the injector holder 54 is received inside the housing 16.

As shown in Fig. 14B, the cover 106 comprises an engaging structure 112. When the injector 44 has been loaded into the injector holder 54 and the injector holder 54 has been pushed in the proximal direction 12 into the housing 16 from the loading position 108 to the delivery position 110, the cover 106 engages the needle shield 48 by means of the engaging structure 112.

When the cover 106 is removed, the needle shield 48 follows the cover 106 and is removed from the needle 46. The cover 106 and the needle shield 48 can then be kept together during an injection process. The above process can then be reversed. Thus, by putting the cover 106 back to the housing 16, the needle shield 48 is also put back onto the injector 44. The injector holder 54 can then be pulled in the distal direction 14 such that the injector holder 54 moves from the delivery position 110 back to the loading position 108. The used injector 44 with the needle shield 48 covering the needle 46 can then be safely lifted out from the injector holder 54. The mechanism 10b in Figs. 13, 14A and 14B enables use with an injector 44 in the form of a syringe or cartridge of traditional design.

Fig. 15 schematically represents a partial proximal view of a flexible strap 114. The strap 114 can be used in various types of injector holders 54, for example in these described above. One end of the strap 114 is secured to the injector holder 54 and the other end is free. In Fig. 15, an injector 44 is held by the injector holder 54. The strap 114 thus lies in an interface between the injector 44 and the injector holder 54. In Fig. 15, the strap 114 is in a relaxed state 116. When the flexible strap 114 is used with the mechanism 10a (as described above), the flexible strap 114 can be used to move the injector 44 from the lower position to the raised position, so that the flexible strap 114 may be used to replace the arrangement of the lifter 58 and the lifer spring 60. Alternatively, the flexible strap 114 can be used with the mechanism 10b.

Fig. 16 schematically represents a further partial proximal view of the strap 114. As illustrated in Fig. 16, the strap 114 can be pulled (to the right in Fig. 16) such that the strap 114 is tensioned. The strap 114 thereby adopts a tensioned state 118. This causes the injector 44 to be lifted up from the injector holder 54 (upwards in Fig. 16). The injector 44 is thus raised by pulling the strap 114. The injector 44 can then be taken out from the mechanism 10b with ease.

Fig. 17A schematically represents a partial cross-sectional side view of a further mechanism 10c. Mainly differences with respect to the mechanism 10a in Figs. 1-12F will be described. In the mechanism 10c in Fig. 17A, the shield holder 70 is connected to the injector holder 54, instead of to the actuating body 24. The shield holder 70 does therefore not rotate together with the actuating body 24.

In Fig. 17A, the shield holder 70 is in the aligned position 90. The shield holder 70 comprises a stop arm 120. The stop arm 120 is movable from a stopping position 122 to a releasing position. In Fig. 17A, the stop arm 120 is in the stopping position 122.

The shield holder 70 of this example further comprises a shield seat 124, a holder arm 126 and a holder spring 128. The needle shield 48 is held in the shield seat 124. The holder arm 126 is arranged between the shield seat 124 and the holder spring 128. The shield seat 124 is proximal of the holder arm 126 and the holder spring 128 is distal of the holder arm 126. The holder spring 128 is here exemplified as a tension coil spring. One end of the holder spring 128 acts on the holder arm 126 and the other end acts on the injector holder 54.

The injector holder 54 of this example further comprises a slot 130. A distal end of the holder arm 126 is guided in the slot 130. The stop arm 120 is pivotally connected to the holder arm 126. In the stopping position 122, the stop arm 120 engages the cam profile 66 of the rotator 64. The rotator 64 is here in the first rotator position 88 and the actuating body 24 is in the first actuating position 28. The holder spring 128 pulls the holder arm 126 and the shield seat 124 in the distal direction 12.

Fig. 17B schematically represents a partial cross-sectional side view of the mechanism 10c in Fig. 17A. In Fig. 17B, the actuating body 24 has moved from the first actuating position 28 to the second actuating position 98 and the rotator 64 has rotated about the longitudinal axis 18 as described above. The position of the mechanism 10c in Fig. 17B may for example correspond to the position of the mechanism 10a in Fig. 8D. The stop arm 120 is gradually released while the rotator 64 is rotating such that the engagement between the stop arm 120 and the cam profile 66 is eventually lost. The stop arm 120 then moves to a releasing position 132. The stop arm 120 thereby no longer prevents movement of the shield holder 70. In the releasing position 132 of the stop arm 120, the holder spring 128 pushes the holder arm 126 to move in the proximal direction 12. The shield seat 124 thereby follows the actuating body 24 in the proximal movement from the first actuating position 28 to the second actuating position 98.

Fig. 17C schematically represents a partial cross-sectional side view of the mechanism 10c in Figs. 17A and 17B. In Fig. 17C, the needle shield 48 is forced from the aligned position 90 to the offset position 100 by magnetic attraction by means of one or more magnets 68 and 72 as described above. The holder arm 126 pivots in the slot 130. The shield seat 124 almost touches the rotator 64. When the user releases the holding force, the actuating body 24 is caused to move from the second actuating position 98 back to the first actuating position 28 as described above. The needle shield 48 is now safely held inside the mechanism 10c and the mechanism 10c is ready for medicament delivery.

Fig. 17D schematically represents a partial cross-sectional side view of the mechanism 10c in Figs. 17A to 17C. In Fig. 17D, the actuating body 24 is moved from the first actuating position 28 to the third actuating position 104 during medicament delivery, as described above. In the third actuating position 104 of the actuating body 24, the holder spring 128 is compressed. The needle shield 48 of the mechanism 10c in Figs. 17A to 17D can be combined with the needle shield 48 of the mechanism 10a in Figs. 1 to 12F.

While the present disclosure has been described with reference to exemplary embodiments, it will be appreciated that the present invention is not limited to what has been described above. For example, it will be appreciated that the dimensions of the parts may be varied as needed. Accordingly, it is intended that the present invention may be limited only by the scope of the claims appended hereto.

## Claims

1. A mechanism (10a, 10c) for receiving an injector (44) having a needle (46) and a needle shield (48) covering the needle (46), the mechanism (10a, 10c) comprising:
- a housing (16) having an injector holder (54) for holding the injector (44);
- a shield holder (70) for holding the needle shield (48), the shield holder (70) being movable relative to the housing (16) between an aligned position (90) for holding the needle shield (48) aligned with the longitudinal axis (18), and an offset position (100) for holding the needle shield (48) offset from the longitudinal axis (18); and
- a rotator (64) rotatable around the longitudinal axis (18) from a first rotator position (88) to a second rotator position (102);
wherein the mechanism (10a, 10c) is configured to cause a movement of the shield holder (70) from the aligned position (90) to the offset position (100) by means of a movement of the rotator (64) from the first rotator position (88) to the second rotator position (102);
**characterized in that**
the mechanism comprises at least one magnet (68) on the rotator and at least one magnet (72) on the shield holder (70),
during the rotation of the rotator (64) about the longitudinal axis (18), the at least one magnet (68) on the rotator (64) is brought into alignment with the at least one magnet (72) on the shield holder (70),
the shield holder (70) is magnetically forced towards the rotator (64) by means of the magnets (68, 72) from the aligned position (90) to the offset position (100) during rotation of the rotator (64) from the first rotator position (88) to the second rotator position (102).

2. The mechanism (10a, 10c) according to claim 1, further comprising an actuating body (24) movable relative to the housing (16) along the longitudinal axis (18) between a first actuating position (28) and a second actuating position (98).

3. The mechanism (10a, 10c) according to claim 2, wherein the actuating body (24) covers the needle (46) in each of the first actuating position (28) and the second actuating position (98).

4. The mechanism (10a, 10c) according to claim 2 or 3, further comprising a guiding member (40) for limiting a movement of the actuating body (24) relative to the housing (16) along the longitudinal axis (18), where the guiding member (40) is arranged to move along the longitudinal axis (18) relative to the housing (16) and is prevented from rotating about the longitudinal axis (18) relative to the housing (16).

5. The mechanism (10a, 10c) according to any of the preceding claims, further comprising a rotator force device (74) arranged to force the rotator (64) in a distal direction (14) along the longitudinal axis (18).

6. The mechanism (10a, 10c) according to a combination of claims 4 and 5, wherein the rotator force device (74) is arranged between the guiding member (40) and the rotator (64).

7. The mechanism (10a, 10c) according to any of the previous claims and claim 2, wherein the rotator (64) is rotatable from the first rotator position (88) to the second rotator position (102) in response to one or more movements of the actuating body (24) between the first actuating position (28) and the second actuating position (98).

8. The mechanism (10a, 10c) according to claim 7, further comprising a cam profile (66) and at least one cam follower (34, 62) arranged to follow the cam profile (66), wherein the rotator (64) is arranged to rotate from the first rotator position (88) to the second rotator position (102) by means of interaction between the at least one cam follower (34, 62) and the cam profile (66) and by means of one or more movements of the actuating body (24) between the first actuating position (28) and the second actuating position (98).

9. The mechanism (10a, 10c) according to claim 8, wherein the cam profile (66) is provided on the rotator (64).

10. The mechanism (10a, 10c) according to claim 8 or 9, wherein one or more of the at least one cam follower (34) is provided on the actuating body (24).

11. The mechanism (10a, 10c) according to any of claims 8 to 10, wherein one or more of the at least one cam follower (62) is provided on the housing (16).

12. The mechanism (10a, 10c) according to any of the previous claims, wherein the one or more magnets (68) on the rotator (64) comprise at least four magnets (68) distributed around an inner surface of the rotator (64) to surround the shield holder (70).

13. The mechanism (10a, 10c) according to any of the previous claims, wherein the magnets (68) on the rotator (64) have poles of different polarities facing the shield holder (70).

## Patentansprüche

1. Mechanismus (10a, 10c) zum Aufnehmen eines Injektors (44), der eine Nadel (46) und einen Nadelschutz (48) aufweist, der die Nadel (46) abdeckt, der Mechanismus (10a, 10c) umfassend:
- ein Gehäuse (16), das einen Injektorhalter (54) zum Halten des Injektors (44) aufweist;
- einen Schutzhalter (70) zum Halten des Nadelschutzes (48), wobei der Schutzhalter (70) relativ zu dem Gehäuse (16) zwischen einer ausgerichteten Position (90) zum Halten des Nadelschutzes (48) in Ausrichtung mit der Längsachse (18) und einer versetzten Position (100) zum Halten des Nadelschutzes (48) von der Längsachse (18) versetzt bewegbar ist; und
- einen Rotator (64), der um die Längsachse (18) von einer ersten Rotatorposition (88) zu einer zweiten Rotatorposition (102) drehbar ist;
wobei der Mechanismus (10a, 10c) konfiguriert ist, um eine Bewegung des Schutzhalters (70) von der ausgerichteten Position (90) zu der versetzten Position (100) mittels einer Bewegung des Rotators (64) von der ersten Rotatorposition (88) zu der zweiten Rotatorposition (102) zu bewirken;
**dadurch gekennzeichnet, dass**
der Mechanismus mindestens einen Magneten (68) an dem Rotator und mindestens einen Magneten (72) an dem Schutzhalter (70) umfasst,
während der Drehung des Rotators (64) um die Längsachse (18) der mindestens eine Magnet (68) an dem Rotator (64) in Ausrichtung mit dem mindestens einen Magneten (72) an dem Schutzhalter (70) gebracht wird,
der Schutzhalter (70) mittels der Magnete (68, 72) aus der ausgerichteten Position (90) in die versetzte Position (100) während der Drehung des Rotators (64) von der ersten Rotatorposition (88) zu der zweiten Rotatorposition (102) zu dem Rotator (64) hin magnetisch gedrängt wird.

2. Mechanismus (10a, 10c) nach Anspruch 1, ferner umfassend einen Betätigungskörper (24), der relativ zu dem Gehäuse (16) entlang der Längsachse (18) zwischen einer ersten Betätigungsposition (28) und einer zweiten Betätigungsposition (98) bewegbar ist.

3. Mechanismus (10a, 10c) nach Anspruch 2, wobei der Betätigungskörper (24) die Nadel (46) in jeder der ersten Betätigungsposition (28) und der zweiten Betätigungsposition (98) abdeckt.

4. Mechanismus (10a, 10c) nach Anspruch 2 oder 3, ferner umfassend ein Führungselement (40) zum Begrenzen einer Bewegung des Betätigungskörpers (24) relativ zu dem Gehäuse (16) entlang der Längsachse (18), wobei das Führungselement (40) angeordnet ist, um sich entlang der Längsachse (18) relativ zu dem Gehäuse (16) zu bewegen und daran gehindert ist, sich um die Längsachse (18) relativ zu dem Gehäuse (16) zu drehen.

5. Mechanismus (10a, 10c) nach einem der vorstehenden Ansprüche, ferner umfassend eine Rotatorkraftvorrichtung (74), die angeordnet ist, um den Rotator (64) in eine distale Richtung (14) entlang der Längsachse (18) zu drängen.

6. Mechanismus (10a, 10c) nach einer Kombination der Ansprüche 4 und 5, wobei die Rotatorkraftvorrichtung (74) zwischen dem Führungselement (40) und dem Rotator (64) angeordnet ist.

7. Mechanismus (10a, 10c) nach einem der vorstehenden Ansprüche und Anspruch 2, wobei der Rotator (64) von der ersten Rotatorposition (88) zu der zweiten Rotatorposition (102) als Reaktion auf eine oder mehrere Bewegungen des Betätigungskörpers (24) zwischen der ersten Betätigungsposition (28) und der zweiten Betätigungsposition (98) drehbar ist.

8. Mechanismus (10a, 10c) nach Anspruch 7, ferner umfassend ein Nockenprofil (66) und mindestens einen Nockenstößel (34, 62), der angeordnet ist, um dem Nockenprofil (66) zu folgen, wobei der Rotator (64) angeordnet ist, um sich von der ersten Rotatorposition (88) zu der zweiten Rotatorposition (102) mittels Wechselwirkung zwischen dem mindestens einen Nockenstößel (34, 62) und dem Nockenprofil (66) und mittels einer oder mehrerer Bewegungen des Betätigungskörpers (24) zwischen der ersten Betätigungsposition (28) und der zweiten Betätigungsposition (98) zu drehen.

9. Mechanismus (10a, 12b, 10c) nach Anspruch 8, wobei das Nockenprofil (66) auf dem Rotator (64) bereitgestellt ist.

10. Mechanismus (10a, 10c) nach Anspruch 8 oder 9, wobei einer oder mehrere des mindestens einen Nockenstößels (34) an dem Betätigungskörper (24) bereitgestellt ist.

11. Mechanismus (10a, 10c) nach einem der Ansprüche 8 bis 10, wobei einer oder mehrere des mindestens einen Nockenstößels (62) an dem Gehäuse (16) bereitgestellt ist.

12. Mechanismus (10a, 10c) nach einem der vorstehenden Ansprüche, wobei der eine oder die mehreren Magnete (68) an dem Rotator (64) mindestens vier Magnete (68) umfassen, die um eine innere Oberfläche des Rotators (64) verteilt sind, um den Schutzhalter (70) zu umgeben.

13. Mechanismus (10a, 10c) nach einem der vorstehenden Ansprüche, wobei die Magnete (68) auf dem Rotator (64) Pole unterschiedlicher Polaritäten aufweisen, die dem Schutzhalter (70) zugewandt sind.

## Revendications

1. Mécanisme (10a, 10c) permettant de recevoir un injecteur (44) ayant une aiguille (46) et une protection d'aiguille (48) couvrant l'aiguille (46), le mécanisme (10a, 10c) comprenant :
- un logement (16) ayant un support d'injecteur (54) permettant de retenir l'injecteur (44) ;
- un support de protection (70) permettant de retenir la protection d'aiguille (48), le support de protection (70) pouvant être déplacé par rapport au logement (16) entre une position alignée (90) permettant de retenir la protection d'aiguille (48) alignée avec l'axe longitudinal (18), et une position décalée (100) permettant de retenir la protection d'aiguille (48) décalée par rapport à l'axe longitudinal (18) ; et
- un rotateur (64) rotatif autour de l'axe longitudinal (18) d'une première position de rotateur (88) à une seconde position de rotateur (102) ;
dans lequel le mécanisme (10a, 10c) est conçu pour amener un déplacement du support de protection (70) de la position alignée (90) à la position décalée (100) au moyen d'un déplacement du rotateur (64) de la première position de rotateur (88) à la seconde position de rotateur (102) ;
**caractérisé en ce que**
le mécanisme comprend au moins un aimant (68) sur le rotateur et au moins un aimant (72) sur le support de protection (70),
pendant la rotation du rotateur (64) autour de l'axe longitudinal (18), l'au moins un aimant (68) sur le rotateur (64) est amené en alignement avec l'au moins un aimant (72) sur le support de protection (70),
le support de protection (70) est forcé magnétiquement en direction du rotateur (64) au moyen des aimants (68, 72) de la position alignée (90) à la position décalée (100) pendant une rotation du rotateur (64) de la première position de rotateur (88) à la seconde position de rotateur (102).

2. Mécanisme (10a, 10c) selon la revendication 1, comprenant en outre un corps d'actionnement (24) déplaçable par rapport au logement (16) le long de l'axe longitudinal (18) entre une première position d'actionnement (28) et une seconde position d'actionnement (98).

3. Mécanisme (10a, 10c) selon la revendication 2, dans lequel le corps d'actionnement (24) couvre l'aiguille (46) dans chacune parmi la première position d'actionnement (28) et la seconde position d'actionnement (98).

4. Mécanisme (10a, 10c) selon la revendication 2 ou 3, comprenant en outre un élément de guidage (40) destiné à limiter un déplacement du corps d'actionnement (24) par rapport au logement (16) le long de l'axe longitudinal (18), là où l'élément de guidage (40) est agencé pour se déplacer le long de l'axe longitudinal (18) par rapport au logement (16) et est empêché d'effectuer une rotation autour de l'axe longitudinal (18) par rapport au logement (16).

5. Mécanisme (10a, 10c) selon l'une quelconque des revendications précédentes, comprenant en outre un dispositif de forçage de rotateur (74) agencé pour forcer le rotateur (64) dans une direction distale (14) le long de l'axe longitudinal (18).

6. Mécanisme (10a, 10c) selon une combinaison des revendications 4 et 5, dans lequel le dispositif de forçage de rotateur (74) est agencé entre l'élément de guidage (40) et le rotateur (64).

7. Mécanisme (10a, 10c) selon l'une quelconque des revendications précédentes et la revendication 2, dans lequel le rotateur (64) peut effectuer une rotation de la première position de rotateur (88) à la seconde position de rotateur (102) en réponse à un ou plusieurs déplacements du corps d'actionnement (24) entre la première position d'actionnement (28) et la seconde position d'actionnement (98).

8. Mécanisme (10a, 10c) selon la revendication 7, comprenant en outre un profil de came (66) et au moins un suiveur de came (34, 62) agencé pour suivre le profil de came (66), dans lequel le rotateur (64) est agencé pour effectuer une rotation de la première position de rotateur (88) à la seconde position de rotateur (102) au moyen d'une interaction entre l'au moins un suiveur de came (34, 62) et le profil de came (66) et au moyen d'un ou plusieurs déplacements du corps d'actionnement (24) entre la première position d'actionnement (28) et la seconde position d'actionnement (98).

9. Mécanisme (10a, 10c) selon la revendication 8, dans lequel le profil de came (66) est fourni sur le rotateur (64).

10. Mécanisme (10a, 10c) selon la revendication 8 ou 9, dans lequel un ou plusieurs parmi l'au moins un suiveur de came (34) sont fournis sur le corps d'actionnement (24).

11. Mécanisme (10a, 10c) selon l'une quelconque des revendications 8 à 10, dans lequel un ou plusieurs parmi l'au moins un suiveur de came (62) sont fournis sur le logement (16).

12. Mécanisme (10a, 10c) selon l'une quelconque des revendications précédentes, dans lequel le ou les aimants (68) sur le rotateur (64) comprennent au moins quatre aimants (68) répartis autour d'une surface interne du rotateur (64) pour entourer le support de protection (70).

13. Mécanisme (10a, 10c) selon l'une quelconque des revendications précédentes, dans lequel les aimants (68) sur le rotateur (64) ont des pôles de polarités différentes faisant face vers le support de protection (70).
